(19) Europäisches Patentamt
European Patent Office
Office européen des brevets

(11) Veröffentlichungsnummer : **0 447 891 B1**

(12) **EUROPÄISCHE PATENTSCHRIFT**

(45) Veröffentlichungstag der Patentschrift : **27.04.94 Patentblatt 94/17**

(51) Int. Cl.$^5$ : **C07D 495/04,** A01N 49/00

(21) Anmeldenummer : **91103547.5**

(22) Anmeldetag : **08.03.91**

(54) **Thieno[2,3-d]pyrimidinderivate.**

(30) Priorität : **19.03.90 DE 4008726**

(43) Veröffentlichungstag der Anmeldung : **25.09.91 Patentblatt 91/39**

(45) Bekanntmachung des Hinweises auf die Patenterteilung : **27.04.94 Patentblatt 94/17**

(84) Benannte Vertragsstaaten : **BE CH DE DK ES FR GB IT LI NL**

(56) Entgegenhaltungen :
**DE-A- 2 654 090**
**US-A- 4 196 207**

(73) Patentinhaber : **BASF Aktiengesellschaft**
**Carl-Bosch-Strasse 38**
**D-67063 Ludwigshafen (DE)**

(72) Erfinder : **Wiesenfeldt, Matthias, Dr.**
**Rosenstrasse 10**
**W-6704 Mutterstadt (DE)**
Erfinder : **Etzbach, Karl-Heinz, Dr.**
**Carl-Bosch-Ring 55**
**W-6710 Frankenthal (DE)**
Erfinder : **Hofmeister, Peter, Dr.**
**Bernard-Humbolt-Strasse 12**
**W-6730 Neustadt (DE)**
Erfinder : **Kuenast, Christoph, Dr.**
**Salierstrasse 2**
**W-6701 Otterstadt (DE)**
Erfinder : **Westphalen, Karl-Otto, Dr.**
**Mausbergweg 58**
**W-6720 Speyer (DE)**

**EP 0 447 891 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft neue Thieno[2,3-d]pyrimidinderivate der allgemeinen Formel I

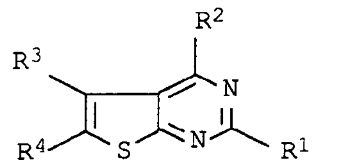

(I)

in der die Substituenten die folgende Bedeutung haben:

$R^1$ Wasserstoff, $C_1$-$C_5$-Alkyl, $C_1$-$C_3$-Chloralkyl, $C_3$-$C_6$-Cycloalkyl, Phenyl oder Benzyl

$R^2$ Fluor, Chlor, Brom, Jod, Hydroxy, Azido, Cyano, Thiocyanato, Mercapto, $C_1$-$C_{20}$-Alkoxy, $C_3$-$C_6$-Alkenyloxy, $C_3$-$C_6$-Alkinyloxy, $C_1$-$C_4$-Alkoxy-$C_2$-$C_6$-alkoxy, Phenoxy-$C_2$-$C_6$-alkoxy, $C_3$-$C_{12}$-Cycloalkoxy, Hydroxy-$C_2$-$C_6$-alkoxy, Chlor-$C_2$-$C_8$-alkoxy, Phenyloxy oder Benzyloxy, Phenylethoxy, Amino-$C_2$-$C_6$-alkoxy, Bis($C_1$-$C_4$-alkyl)amino-$C_2$-$C_6$-alkoxy, Hydroxy-$C_1$-$C_4$-alkyl-amino-$C_2$-$C_6$-alkoxy, $C_1$-$C_{18}$-Alkylthio, $C_3$-$C_6$-Cycloalkylthio , $C_3$-$C_6$-Alkenylthio, $C_3$-$C_6$-Alkinylthio, Hydroxy-$C_2$-$C_6$-alkylthio, Bis-$C_1$-$C_3$-alkylamino-$C_2$-$C_6$-alkylthiol Carboxy-$C_1$-$C_4$-alkylthio, $C_1$-$C_3$-Alkoxycarbonyl-$C_1$-$C_2$-alkylthio, Furylmethylthio, Phenylthio oder Benzylthio, $C_1$-$C_4$-Alkoxycarbonylmethyl, Carboxymethyl, Bis-($C_1$-$C_4$-alkoxycarbonyl)methyl, α-$C_1$-$C_4$-Alkoxycarbonyl-α-cyanomethyl, Dicyanomethyl, Bis(carboxy)methyl, α-Cyano-α-phenyl-methyl, Pyrrolidino, Piperidino, Morpholino, Hexamethylenimino, N-Methylpiperazino, N-Ethylpiperazino, Imidazolyl, Pyrazolyl, Triazolyl, Tetrazolyl, Thiomorpholino, Hydrazino, $C_1$-$C_3$-Alkylhydrazino, Bis-($C_1$-$C_3$-alkyl)hydrazino, Phenylhydrazino, Hydroxylamino, Morpholin-(1)-yl-amino, Hexamethylenimino-(1)-yl-amino, oder ein Rest $NR^5R^6$, worin $R^5$ und $R^6$ unabhängig voneinander Wasserstoff, Amino oder $C_1$-$C_{20}$-Alkyl, $C_3$-$C_{20}$-Alkenyl, $C_3$-$C_6$-Alkinyl, $C_3$-$C_{12}$-Cycloalkyl, $C_3$-$C_6$-Cycloalkyl-$C_1$-$C_4$-alkyl, Hydroxy-$C_2$-$C_6$-Alkyl, Amino-$C_2$-$C_{12}$-alkyl, Bis-$C_1$-$C_4$-alkylamino-$C_2$-$C_6$-alkyl, Piperazin-(1)-yl-$C_2$-$C_4$-alkyl, Morpholin-(1)-yl-$C_2$-$C_4$-alkyl, $C_1$-$C_3$-Alkoxy-$C_1$-$C_4$-alkyl, Phenoxy-$C_4$-$C_6$-Alkyl, Benzyloxy-$C_2$-$C_6$-alkyl, Hydroxy-$C_2$-$C_3$-alkoxy-$C_2$-$C_6$-alkyl, Hydroxy-$C_2$-$C_4$-alkylamino-$C_2$-$C_4$-alkyl, $C_2$-$C_3$-Alkoxy-$C_2$-$C_3$-alkoxy-$C_2$-$C_6$-alkyl, Phenoxy-$C_2$-$C_4$-alkoxy-$C_2$-$C_4$- alkyl, Bis-hydroxymethyl-$C_1$-$C_3$-alkyl, Cyano-$C_2$-$C_{10}$-alkyl, Carboxy-$C_2$-$C_{10}$-alkyl, $C_1$-$C_3$-Alkoxy-$C_2$-$C_3$alkoxy-$C_2$-$C_3$-alkoxy-$C_2$-$C_3$-alkyl, Phenyl, Phenyl-$C_1$-$C_4$-alkyl, $C_3$-$C_{12}$-Cycloalkyl, Pyridyl, Furyl, Thienyl, Methylpyridyl, Pyridylmethyl, Furylmethyl, Thienylmethyl oder Adamantyl bedeuten oder gemeinsam mit dem Stickstoffatom einen heterocyclischen Rest mit 3 bis 8 Ringatomen bilden, mit der Maßgabe, daß $R^2$ nicht Phenoxy-$C_2$-$C_3$-alkylamino bedeutet,

$R^3$ Chlor, Brom, Hydroxy oder Mercapto mit der Maßgabe, daß nicht gleichzeitig $R^2$ Hydroxy und $R^3$ Brom bedeutet und daß nicht gleichzeitig $R^3$ Hydroxy und $R^4$ Alkoxycarbonyl oder Carboxy bedeutet,

$R^4$ Wasserstoff, $C_1$-$C_6$-Alkyl, $C_3$-$C_6$-Mono-, Di- oder Trihalogenalkyl, Phenyl, Cyano, Formyl, Hydroxyiminomethyl, Carboxy, Alkoxycarbonyl, Aminocarbonyl oder ein Rest der Formel

$$CH{=}X$$

worin X für $C_1$-$C_{20}$-Alkylimino, Phenylimino, Carboxymethylen, α-$C_1$-$C_4$-Alkyl-α-carboxymethylen, α-Phenyl-α-carboxymethylen, Dicarboxymethylen, Cyanomethylen, α-$C_1$-$C_4$-Alkyl-α-cyanomethylen, α-Phenyl-α-cyanomethylen, Dicyanomethylen, Formylmethylen, α-$C_1$-$C_4$-Alkyl-α-formylmethylen, α-Phenyl-α-formylmethylen, Diformylmethylen, $C_1$-$C_4$-Alkoxycarbonylmethylen, α-$C_1$-$C_4$-Alkyl-α-$C_1$-$C_4$-Alkoxycarbonylmethylen, $C_1$-$C_4$-Di-alkoxycarbonylmethylen, α-Cyano-α-$C_1$-$C_4$-alkoxycarbonylmethylen oder α-Phenyl-α-$C_1$-$C_4$-alkoxycarbonylmethylen steht,

wobei, wenn $R^1$ und/oder $R^2$ Phenyl bedeuten oder enthalten, dieser Phenylrest bis zu 3 gleiche oder verschiedene Substituenten, wie $C_1$-$C_4$-Alkyl, $C_1$-$C_4$-Alkoxy, Halogen, Nitro, Amino, Hydroxy, Hydroxy-$C_1$-$C_4$-alkyl, Cyano, Carboxy, Aminocarbonyl, $C_1$-$C_4$-Alkoxycarbonyl, $C_1$-$C_4$-Acyloxy, $C_2$-$C_6$-Acylamino, $C_1$-$C_4$-Alkylamino, $C_1$-$C_3$-Acyl, Carboxy-$C_1$-$C_2$-alkyl, Di-$C_1$-$C_4$-alkylamino sowie Trifluormethyl tragen kann, sowie die Tautomeren dieser Thieno[2,3-d]pyrimidinderivate und die nutzpflanzenverträglichen Salze dieser Thieno[2,3-d]pyrimidinderivate und ihrer Tautomeren.

Außerdem betrifft die Erfindung die Herstellung der Verbindungen I, Mittel zur Bekämpfung von Schadpflanzen und Schadinsekten sowie zur Regulierung des Wachstums von Nutzpflanzen, die Verbindungen I als Wirkstoffe enthalten, sowie die Verwendung der Verbindungen I als Herbizide, Schädlingsbekämpfungsmittel oder Wachstumsregulatoren.

In der DE-A-26 54 090 und der US-A 4,196,207 sind Thieno[2,3-d]-pyrimidinderivate mit fungiziden, bakteriziden, antiviralen, wachstumsregulierenden und insektiziden bzw. akariziden Eigenschaften beschrieben,

die in 5-Position auch halogensubstituiert sein können. In der Beschreibung und in den Beispielen werden jedoch nur Wasserstoff und Methyl als Substituenten in der 5-Position genannt. Die Wirksamkeit dieser Substanzen als Herbizide, Schädlingsbekämpfungsmittel und wachstumsregulierende Mittel ist unbefriedigend. Weiterhin sind aus den Arbeiten von M.M. Robba et al. (C.R. Acad. Sc. Paris (1968), 267, 697-700) und N.V. Kaplina et al. (Pharm. Chem. Journal (1987), 21 (2) 126-129) Thieno(2,3-d)pyrimidinderivate bekannt, in denen in 4-Stellung Hydroxy und in 5-Stellung Brom steht. Kaplina et al. haben diese Verbindungen auf antivirale Aktivität untersucht. Ferner haben S. Kohra et al. (J.Het. Chem., (1988), 25, 959-968) das 4,5-Dihydroxy-6-methoxycarbonyl-2-phenyl-thieno[2,3-d]pyrimidin beschrieben. Eine Methode zur Herstellung von 4-Oxo-, 4-Thio- oder 4-Imino-thieno[2,3-d]pyrimidinen wird in der IN-151 496 beansprucht.

Der Erfindung lag die Aufgabe zugrunde, Thieno[2,3-d]pyrimidinderivate mit herbiziden und verbesserten wachstumsregulierenden sowie verbesserten insektiziden Wirkungen bereitzustellen.

Demgemäß wurden die eingangs definierten Thieno[2,3-d]pyrimidinderivate I gefunden. Die Verbindungen I weisen bemerkenswerte insektizide, herbizide und wachstumsregulierende Eigenschaften auf.

Die Verbindungen I, in denen $R^2$ und/oder $R^3$ für die Hydroxy oder die Mercaptogruppe stehen, bilden Keto-Enol-Tautomere. Beide Formen gehören ebenso zu den Verbindungen nach der Erfindung wie die pflanzenverträglichen Salze, z.B. von Mineralsäuren, wie Salzsäure, Schwefelsäure oder Phosphorsäure, oder von organischen Säuren, wie Essigsäure, Malonsäure, Bernsteinsäure oder Fumarsäure.

Unter den Verbindungen I sind diejenigen bevorzugt, in denen die Substituenten die folgende Bedeutung haben:

$R^1$ Wasserstoff, $C_1$-$C_5$-Alkyl, $C_1$-$C_3$-Chloralkyl, $C_3$-$C_6$-Cycloalkyl, Phenyl oder Benzyl

$R^2$ Hydroxy, Fluor, Chlor, Brom, Azido, Cyano, Thiocyanato, $C_1$-$C_{20}$-Alkoxy, $C_3$-$C_6$-Alkenyloxy, $C_3$-$C_6$-Alkinyloxy, $C_1$-$C_4$-Alkoxy-$C_2$-$C_6$-alkoxy, Phenoxy-$C_2$-$C_6$-alkoxy, $C_3$-$C_{12}$-Cycloalkoxy, Hydroxy-$C_2$-$C_6$-alkoxy, Chlor-$C_2$-$C_8$-alkoxy, Phenyloxy oder Benzyloxy, Phenylethoxy, Amino-$C_2$-$C_6$-alkoxy, Bis($C_1$-$C_4$-alkyl)amino-$C_2$-$C_6$-alkoxy, Hydroxy-$C_1$-$C_4$-alkylamino-$C_2$-$C_6$-alkoxy, Mercapto, $C_1$-$C_{18}$-Alkylthio, $C_3$-$C_6$-Cycloalkylthio, $C_3$-$C_6$-Alkenylthio, $C_3$-$C_6$-Alkinylthio, Hydroxy-$C_2$-$C_6$-alkylthio, Bis-$C_1$-$C_3$-alkylamino-$C_2$-$C_6$-alkylthio, Carboxy-$C_1$-$C_4$-alkylthio, $C_1$-$C_3$-Alkoxycarbonyl-$C_1$-$C_2$-alkylthio, Furylmethylthio, Phenylthio oder Benzylthio, $C_1$-$C_4$-Alkoxycarbonylmethyl, Carboxymethyl, Bis-($C_1$-$C_4$-alkoxycarbonyl)methyl, α-$C_1$-$C_4$-Alkoxycarbonyl-α-cyanomethyl, Dicyanomethyl, Bis(carboxy)methyl, α-Cyano-α-phenyl-methyl, Pyrrolidino, Piperidino, Morpholino, Hexamethylenimino, N-Methylpiperazino, N-Ethylpiperazino, Imidazolyl, Pyrazolyl, Triazolyl, Tetrazolyl, Thiomorpholino, Hydrazino, $C_1$-$C_3$-Alkylhydrazino, Bis-($C_1$-$C_3$-alkyl)hydrazino, Phenylhydrazino, Hydroxylamino, Morpholin-(1)-yl-amino, Hexamethylenimino-(1)-yl-amino, einen Rest $NR^5R^6$, worin $R^5$ und $R^6$ unabhängig voneinander für Wasserstoff, $C_1$-$C_{20}$-Alkyl, $C_3$-$C_{20}$-Alkenyl, $C_3$-$C_6$-Alkinyl, $C_3$-$C_{12}$-Cycloalkyl, $C_3$-$C_6$-Cycloalkyl-$C_1$-$C_4$-alkyl, Hydroxy-$C_2$-$C_6$-Alkyl, Amino-$C_2$-$C_{12}$-alkyl, Bis-$C_1$-$C_4$-alkylamino-$C_2$-$C_6$-alkyl, Piperazin-(1)-yl-$C_2$-$C_4$-alkyl, Morpholin-(1)-yl-$C_2$-$C_4$-alkyl, $C_1$-$C_3$-Alkoxy-$C_1$-$C_4$-alkyl, Phenoxy-$C_4$-$C_6$-Alkyl, Benzyloxy-$C_2$-$C_6$-alkyl, Hydroxy-$C_2$-$C_3$-alkoxy-$C_2$-$C_6$-alkyl, Hydroxy-$C_2$-$C_4$-alkylamino-$C_2$-$C_4$-alkyl, $C_2$-$C_3$-Alkoxy-$C_2$-$C_3$-alkoxy-$C_2$-$C_6$-alkyl, Phenoxy-$C_2$-$C_4$-alkoxy-$C_2$-$C_4$-alkyl, Bis-hydroxymethyl-$C_1$-$C_3$-alkyl, Cyano-$C_2$-$C_{10}$-alkyl, Carboxy-$C_2$-$C_{10}$-alkyl, $C_1$-$C_3$-Alkoxy-$C_2$-$C_3$alkoxy-$C_2$-$C_3$-alkoxy-$C_2$-$C_3$-alkyl, Phenyl, Phenyl-$C_1$-$C_4$-alkyl, $C_3$-$C_{12}$-Cycloalkyl, Pyridyl, Furyl, Thienyl, Methylpyridyl, Pyridylmethyl, Furylmethyl, Thienylmethyl oder Adamantyl stehen,
wobei, wenn $R^1$ und/oder $R^2$ Phenyl bedeuten oder enthalten, dieser Phenylrest bis zu 3 gleiche oder verschiedene Substituenten, wie $C_1$-$C_4$-Alkyl, $C_1$-$C_4$-Alkoxy, Halogen, Nitro, Amino, Hydroxy, Hydroxy-$C_1$-$C_4$-alkyl, Cyano, Carboxy, Aminocarbonyl, $C_1$-$C_4$-Alkoxycarbonyl, $C_1$-$C_4$-Acyloxy, $C_2$-$C_6$-Acylamino, $C_1$-$C_4$-Alkylamino, $C_1$-$C_3$-Acyl, Carboxy-$C_1$-$C_2$-alkyl, Di-$C_1$-$C_4$-alkylamino sowie Trifluormethyl und insbesondere Fluor, Chlor, Brom, Jod, Methyl, Ethyl, Amino, Hydroxy, Methoxy, Ethoxy, Acetylamino oder Acetyl tragen kann,

$R^3$ Chlor, Brom oder Hydroxy

$R^4$ Wasserstoff, Methyl, $C_1$-$C_2$-Mono- oder Dichloralkyl, Cyano, Carboxy, $C_1$-$C_4$-Alkoxycarbonyl, Aminocarbonyl oder CH=X, worin X für $C_1$-$C_{20}$-Alkylimino, Phenylimino, Carboxymethylen, α-$C_1$-$C_4$-Alkyl-α-carboxymethylen, α-Phenyl-α-carboxymethylen, Dicarboxymethylen, Cyanomethylen, α-$C_1$-$C_4$-Alkyl-α-cyanomethylen, α-Phenyl-α-cyanomethylen, Dicyanomethylen, Formylmethylen, α-$C_1$-$C_4$-Alkyl-α-formylmethylen, α-Phenyl-α-formylmethylen, Diformylmethylen, $C_1$-$C_4$-Alkoxycarbonylmethylen, α-$C_1$-$C_4$-Alkyl-α-$C_1$-$C_4$-Alkoxycarbonylmethylen, $C_1$-$C_4$-Dialkoxycarbonylmethylen, α-Cyano-α-$C_1$-$C_4$-alkoxycarbonylmethylen oder α-Phenyl-α-$C_1$-$C_4$-alkoxycarbonylmethylen steht.

Für den erfindungsgemäßen Zweck bevorzugte Verbindungen sind in Tabelle 1 aufgeführt, wobei die Verbindungen 4,5-Dichloro-6-methyl- (Nr. 12), 4-n-Propylamino-5-chloro- (Nr. 144), 4-n-Butylamino-5-chloro- (Nr. 146), 4-sek.Butylamino-5-chloro- (Nr. 147), 4-n-Hexylamino-5-chloro- (Nr. 154), 4-n-Heptylamino-5-chloro- (Nr. 155), 4-n-Octylamino-5-chloro- (Nr. 156), 4-n-Nonylamino-5-chloro- (Nr. 157), 4-(2-Methyl-hept-6-ylamino)-5-chloro- (Nr. 168), 4-(1-Ethoxyprop-3-ylamino)-5-chloro- (Nr. 185), 4-Benzylamino-5-chloro- (Nr. 275), 4-Phenylethylamino-5-chloro- (Nr. 282), 4-(Prop-1-in-3-ylamino)-5-chloro- (Nr. 293) und 4-(Hept-6-ylamino)-5-chloro-thieno[2,3-d]pyrimidin (Nr. 395) besonders bevorzugt sind.

Die erfindungsgemäßen Verbindungen I können z.B. hergestellt werden, indem man Thiophenderivate der allgemeinen Formel II

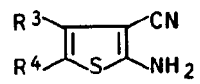

(II)

mit einem Dialkylamid III

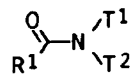

(III)

in dem $T^1$ und $T^2$ unabhängig voneinander für $C_1$-$C_4$-Alkyl oder zusammen mit dem Amidstickstoff für einen 5 bis 7 gliedrigen gesättigten Heterocyclus stehen, in Gegenwart von 2 bis 20 mol Phosphorylchlorid oder Phosphorylbromid, bezogen auf 1 mol II, zu Thieno(2,3-d)pyrimidinderivaten der allgemeinen Formel Ia

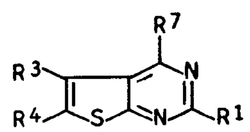

(Ia)

in der $R^7$ für Chlor oder Brom steht, umsetzt und in diesen gegebenenfalls $R^7$ in an sich üblicher Weise gegen andere Nucleophile Reste $R^2$ austauscht.

Geeignete Dialkylamide III sind zum Beispiel N,N-Dimethylformamid, N,N-Dimethylacetamid, N,N-Diethylformamid, N,N-Diethylacetamid, N,N-Dimethylpropionamid und N,N-Dimethylbenzoesäureamid.

Wichtig für das erfindungsgemäße Verfahren ist die Verwendung eines Überschusses von Phosphorylchlorid bzw. Phosphorylbromid bezogen auf das eingesetzte Thiophenderivat II. Es kann auch vorteilhaft sein, die Reaktion in dem geeigneten Phosphorylhalogenid als Lösungsmittel durchzuführen. Man verwendet bevorzugt 2 bis 6 mol Phosphorylhalogenid pro Mol II.

Das Molverhältnis von Thiophenderivat II zu N,N-Dialkylamid III beträgt in der Regel 1:1 bis 1:5.

Zweckmäßig wird die Reaktion in einem inerten Lösungsmittel, wie Chlorbenzol, Nitrobenzol, Benzoesäuremethylester, Methylenchlorid, Dichlorbenzol, Trichlorbenzol, Benzoesäureethylester, Chloroform, Tetrachlorkohlenstoff, eines der oben beschriebenen N,N-Dialkylamide, Trichlorethan, Hexamethylphosphorsäuretriamid (HMPT) oder Tetrachlorethylen, durchgeführt. Bevorzugte Lösungsmittel sind Phosphorylchlorid, Phosphorylbromid oder eines der oben beschriebenen N,N-Dialkylamide.

Die Reaktion verläuft ab etwa 20°C mit ausreichender Geschwindigkeit. Bei Temperaturen von über 150°C sinkt die Spezifität der Reaktion. Vorzugsweise führt man die Reaktion in einem Temperaturbereich von 50 bis 110°C durch.

Die Verwendung von katalytischen Mengen einer Lewis Säure, wie Kaliumchlorid, Natriumchlorid, Eisen-(III)-chlorid, Aluminiumchlorid, Zinkchlorid, Zinnchlorid, Antimonpentafluorid, Bortrichlorid, Titantetrachlorid, oder eines basischen Katalysators wie N,N-Dimethylanilin oder N,N-Diethylanilin, kann zu einer Steigerung der Reaktionsgeschwindigkeit und einer Erhöhung der Ausbeute führen.

Die so erhaltenen Verbindungen der allgemeinen Formel Ia kann man nach bekannten Methoden (The Chemistry of Heterocyclic Compounds, "The pyrimidines", ed. D. J. Brown, J. Wiley &Sons, New York, London, Vol. 16, (1962); Vol. 16, Suppl. 1, (1970); Vol. 16, Suppl. 2 (1985)) durch den Austausch des Halogenatoms in Position 4 gegen andere nucleophile Reste $R^2$ in die korrespondierenden Verbindungen I umwandeln.

Ein weiteres Verfahren zur Herstellung der Thienopyrimidinderivate der allgemeinen Formel I nach Anspruch 1, ist dadurch gekennzeichnet, daß man Verbindungen der allgemeinen Formel IV

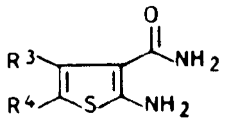

(IV)

in der die Substituenten $R^3$ und $R^4$ die in Anspruch 1 angegebene Bedeutung haben, in an sich üblicher Weise mit einem Säureanhydrid, das mindestens einen Rest $R^1$-CO- enthält, oder einer Carbonsäure $R^1$-COOH, oder einem Addukt aus einer Carbonsäure $R^1$-COOH und einer Lewissäure, wobei $R^1$ jeweils die angegebene Bedeutung hat, zu Verbindungen der allgemeinen Formel Ib

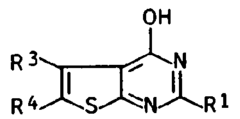

(Ib)

umsetzt, diese mit einem Phosphorylhalogenid in Verbindungen der allgemeinen Formel Ia gemäß Anspruch 2 überführt und in diesen gegebenenfalls $R^7$ in an sich üblicher Weise gegen andere nucleophile Reste $R^2$ austauscht.

In bestimmten Fällen ist es zweckmäßig, die Umsetzung von IV zu Ib in zwei Schritten durchzuführen, d.h. die als Zwischenprodukt auftretenden Verbindungen der allgemeinen Formel VI

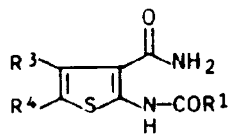

(VI)

zu isolieren.

In der Regel setzt man das Säureanhydrid oder das Addukt in Mengen von 100 bis 500 mol-%, bevorzugt 100 bis 300 mol-%, bezogen auf die Verbindungen IV, ein.

Die Carbonsäureanhydride, die mindestens einen Rest $R^1$-CO- enthalten, können aus zwei Carbonsäuren $R^1$-COOH, wie Pivalinsäure, Propionsäure oder Essigsäure; oder aus einer Carbonsäure $R^1$-COOH und einer Oxosäure, wie Phosphorsäure oder Schwefelsäure, aufgebaut sein.

Bevorzugte Carbonsäuren $R^1$-COOH sind solche mit 1 bis 4 Kohlenstoffatomen, insbesondere Ameisensäure und Essigsäure.

Außerdem kommen auch Addukte aus einer Carbonsäure $R^1$-COOH und einer Lewissäure, wie Zinkchlorid, Bortrifluorid oder Titantetrachlorid, in Betracht.

Die Umsetzung von IV zu Ib erfolgt vorteilhaft in inerten Lösungsmitteln, wie N,N-Dialkylamiden, von denen N,N-Dimethylformamid und Dimethylacetamid besonders bevorzugt sind, N-Methylpyrrolidon, N,N-Dimethylpropylenharnstoff oder Hexamethylphosphorsäuretriamid, bei Temperaturen von (-10) bis 150°C, vorzugsweise 20 bis 120°C, insbesondere 80 bis 120°C.

Für den Fall, daß man die intermediär auftretenden Verbindungen VI isolieren will, wählt man vorzugsweise Reaktionstemperaturen zwischen (-10) und 80°C.

In der Regel gibt man eine Base wie Triethylamin, N-Methylpyrrolidon oder N,N-Dimethylanilin, in einem 1- bis 10-fachen Überschuß, bevorzugt 1- bis 5-fachen Überschuß zu, bezogen auf das Carbonsäureanhydrid, die Carbonsäure oder das Carbonsäure-Lewissäure-Addukt.

Der Zusatz eines wasserentziehenden Reagenzes, wie Dicyclohexylcarbodiimid, oder eines Vilsmeier-Reagenzes kann die Reaktion beschleunigen und die Ausbeute an Ib erhöhen.

Die Hydroxygruppe in 4-Stellung der Verbindungen Ib kann man anschließend nach an sich bekannten Methoden beispielsweise mit Phosphorylchlorid oder Phosphorylbromid gegen Chlor oder Brom austauschen.

Gegegenenfalls kann man das Chlor bzw. Brom in 4-Stellung nach an sich bekannten Methoden gegen andere nucleophile Reste $R^2$ substituieren.

Auch durch die Umsetzung der Verbindungen II mit Orthoestern (V) gemäß Anspruch 4 zu den Thiophenderivaten VI und anschließende Cyclisierung in Gegenwart eines geeigneten Nucleophils können die erfindungsgemäßen Thieno[2,3-d]pyrimidinderivate I erhalten werden.

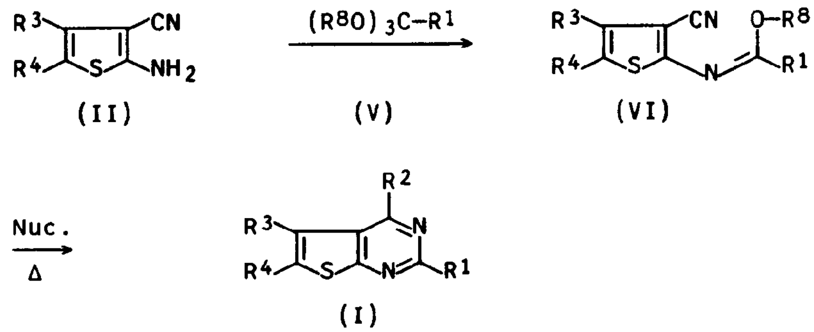


(II) (V) (VI)

(I)

Die zuletzt beschriebene Reaktionsfolge ist an sich aus der DE-A 26 54 090 bekannt. Die Thiophenderivate II und IV kann man nach den in der EP-A1 0193 885 beschriebenen Vorschriften erhalten.

Zur Darstellung von pflanzenverträglichen Salzen kann man die Thieno(2,3-d)pyrimidinderivate I mit üblicherweise verwendeten Salzbildnern, wie Chlorwasserstoffsäure, Bromwasserstoffsäure, Jodwasserstoffsäure, Schwefelsäure, Phosphorsäure, Ameisensäure, Essigsäure, Oxalsäure, Benzolsulfonsäure, p-Toluolsulfonsäure, Dodecylbenzolsulfonsäure, Methylbromid, Dimethylsulfat oder Diethylsulfat im Temperaturbereich von 0 bis 150°C, vorzugsweise 20 bis 120°C, umsetzen.

Die Salzbildung wird zweckmäßigerweise in einem Lösungs- oder Verdünnungsmittel durchgeführt. Hierzu eignen sich beispielsweise aliphatische Kohlenwasserstoffe, wie n-Pentan, n-Hexan oder Petrolether, aromatische Kohlenwasserstoffe, wie Benzol, Toluol oder Xylole, Benzin oder Ether, wie Diethylether, Methyl-tert.-butylether, Tetrahydrofuran oder Dioxan, ferner Ketone, wie Aceton, Methylethylketon oder Methylisopropylketon, sowie halogenierte Kohlenwasserstoffe, wie Chlorbenzol, Methylenchlorid, Ethylenchlorid, Chloroform und Tetrachlorethylen. Auch Gemische dieser Lösungsmittel können verwendet werden.

Zur Herstellung der Salze der Verbindungen I setzt man die Ausgangsstoffe üblicherweise in stöchiometrischem Verhältnis ein. Ein Überschuß der einen oder anderen Komponente kann von Vorteil sein.

Die erfindungsgemäßen Herbizide, Wachstumsregulatoren und Schädlingsbekämpfungsmittel können beispielsweise direkt versprühbare Lösungen, Pulver, Suspensionen, auch hochprozentige wäßrige, ölige oder sonstige Suspensionen, wäßrige oder ölige Dispersionen, Emulsionen, Pasten, Stäubemittel, Streumittel oder Granulate sein und durch Versprühen, Vernebeln, Verstäuben, Verstreuen oder Gießen angewendet werden. Die Anwendungformen richten sich nach den Verwendungszwecken; sie sollten in jedem Fall eine möglichst feine Verteilung der erfindungsgemäßen Wirkstoffe gewährleisten.

Als inerte Trägerstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Oberflächenaktive Stoffe erleichtern die feine Verteilung. Als solche kommen Alkali-, Erdalkali-, Ammoniumsalze von Ligninsulfonsäure, Naphthalinsulfonsäure, Phenolsulfonsäure, Dibutylnaphthalinsulfonsäure; Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Alkylarylpolyglykolether, wie ethoxyliertes Isooctylphenol, Octylphenol, Nonylphenol, Tributylphenylpolyglykolether, Fettalkoholethoxylate, wie Isotridecylethoxylat und wie ethoxyliertes Rizinusöl, Kondensationsprodukte aus Ethylenoxid und Propylenoxid, wie ethoxyliertes Polyoxypropylen, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten üblicherweise zwischen 0,01 und 99 Gew.%, vorzugsweise zwischen 0,1 und 70 Gew.-%, Wirkstoff. Die Wirkstoffe werden zweckmäßig in einer Reinheit von 80 % bis 99,9 %, vorzugsweise 90 % bis 99 % (nach NMR-Spektrum) eingesetzt

Die erfindungsgemäßen Verbindungen I können beispielsweise wie folgt formuliert werden:

I. Man vermischt 90 Gewichtsteile der Verbindung Nr. 431 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 154 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 155 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 168 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 385 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 274 werden mit 97 Gewichtsteilen feinteiligem Kaolin vermischt oder vermahlen. Man erhält auf diese Weise ein Stäubemittel, das 3 Gew.% des Wirkstoffs enthält.

VII. 30 Gewichtsteile des Wirkstoffs Nr. 281 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt oder vermahlen. Man erhält auf diese Weise eine Aufbereitung des Wirkstoffs mit guter Haftfähigkeit.

VIII. 20 Gewichtsteile des Wirkstoffs Nr. 292 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt oder vermahlen. Man erhält eine stabile ölige Dispersion.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

In Anbetracht der Vielseitigkeit der Applikationsmethoden können die erfindungsgemäßen Verbindungen bzw. sie enthaltende Mittel in einer großen Zahl von Kulturpflanzen zur Beseitigung unerwünschter Pflanzen eingesetzt werden.

Zur Verbreiterung des Wirkungsspektrums und zur Erzielung synergistischer Effekte können die erfindungsgemäßen Verbindungen 1 mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3, 1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide in Betracht.

Außerdem kann es von Nutzen sein, die Verbindungen I allein oder in Kombination mit anderen Herbiziden

auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff bei Anwendung als Herbizide betragen je nach Bekämpfungsziel, Jahreszeit, Zielpflanzen und Wachstumsstadium 0,001 bis 5, vorzugsweise 0,01 bis 3 kg/ha aktive Substanz.

Die Verbindungen der Formel I können praktisch alle Entwicklungsstadien einer Pflanze verschiedenartig beeinflussen und werden deshalb auch als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze, und von der Jahreszeit,

c) von dem Applikationsort und -verfahren (Samenbeize, Bodenbehandlung, Blattapplikation oder Stamminjektion bei Bäumen)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Die erfindungsgemäß zu verwendenden Wachstumsregulatoren der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

Die Thieno[2,3-d]pyrimidinderivate der Formel I sind auch geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae,

Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea, beispielsweise Spinnentiere (Acarina) wie Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden, beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Bei der Verwendung der Verbindungen I als Insektizide können die Wirkstoffkonzentrationen in den anwendungsfertigen Zubereitungen in größeren Bereichen variiert werden.

Im allgemeinen liegen sie zwischen 0,01 und 95 Gew.-%, vorzugsweise zwischen 0,1 und 70 Gew.-%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen zweckmäßig 0,001 bis 5, vorzugsweise 0,01 bis 3 kg/ha.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der Tabelle I mit ihren Schmelzpunkten aufgeführt.

Beispiel 1

70 g 2-Amino-3-cyano-4-oxo-4,5-dihydro-thiophen wurden bei Raumtemperatur in 250 ml Phosphorylchlorid ($POCl_3$) eingetragen. Zu diesem Reaktionsgemisch wurden innerhalb von 20 Minuten 38 ml Dimethylformamid (DMF) zugetropft und die Lösung anschließend eine Stunde bei Raumtemperatur und 2,5 Stunden bei 75°C gerührt. Danach wurde das Gemisch auf 2,5 kg Eis gegeben und der Niederschlag abgesaugt, mit Wasser gewaschen und im Vakuum bei 50°C getrocknet. Man erhielt 95,8 g (94 % d. Th.) der Verbindung der Formel

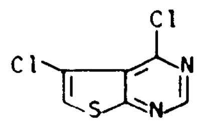

vom Schmelzpunkt 161-162°C.

Beispiel 2

69 g 2-Aldoximo-2-amino-3-cyano-4-oxo-4,5-dihydro-thiophen wurden bei Raumtemperatur in 135 ml Phosphorylchlorid ($POCl_3$) eingetragen. Zu diesem Reaktionsgemisch wurden innerhalb von 20 Minuten 20 ml Dimethylformamid (DMF) zugetropft und die Lösung anschließend eine Stunde bei 20°C und 3 Stunden bei 75°C gerührt. Danach wurde das Gemisch auf 1 kg Eis gegeben, der Niederschlag abgesaugt, mit Wasser gewaschen und im Vakuum bei 50°C getrocknet. Man erhielt 50,2 g (81 % d. Th.) der Verbindung der Formel

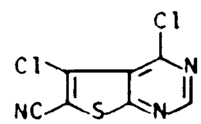

vom Schmelzpunkt 155-157°C.

Beispiel 3

Variante a:

In 150 ml tert.-Butanol wurden 2,5 g elementares Natrium gelöst. Hierzu wurden 10,2 g 4,5-Dichlor-thieno[2,3-d]pyrimidin (Beispiel 1) gegeben. Das Reaktionsgemisch wurde eine Stunde bei 20°C und 4 Stunden bei 50°C gerührt. Anschließend wurde das Reaktionsgemisch in 500 ml Wasser eingerührt und mit Salzsäure auf pH 7 eingestellt. Der Niederschlag wurde abgesaugt, mit Wasser gewaschen und im Vakuum bei 50°C getrocknet. Man erhielt 7,6 g (37 % d. Th.) der Verbindung der Formel

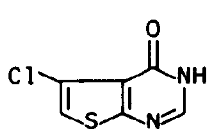

vom Schmelzpunkt 245-248°C.

Variante b:

In 67 ml DMF wurden 20,5 g 4,5-Dichlor-thieno[2,3-d]pyrimidin (Beispiel 1) gelöst. Hierzu wurden bei Raumtemperatur 17,6 g 25 gew.-%ige wäßrige Natronlauge getropft. Das Reaktionsgemisch wurde 16 Stunden bei Raumtemperatur gerührt. Anschließend wurden weitere 17,6 g 25 gew.-%ige wäßrige Natronlauge zugetropft. Nach 24 Stunden Rühren bei 20°C wurde das Reaktionsgemisch auf 500 ml Wasser gegeben und mit Salzsäure auf pH 7 eingestellt. Der Niederschlag wurde abgesaugt, mit Wasser gewaschen und im Vakuum

bei 50°C getrocknet. Man erhielt 16,4 g (88 % d. Th.) der Verbindung, welche mit dem nach Variante a erhaltenen Produkt identisch ist.

Beispiel 4

20,5 g 4,5-Dichlor-thieno[2,3-d]pyrimidin (Beispiel 1) wurden in 150 ml Methanol suspendiert. Hierzu wurden 13,3 g Natriummethanolat gegeben. Danach wurde das Reaktionsgemisch eine Stunde bei Raumtemperatur und eine weitere Stunde bei 50-60°C gerührt. Anschließend wurde das Reaktionsgemisch auf 500 ml Wasser gegeben, der Niederschlag abgesaugt, mit Wasser gewaschen und im Vakuum bei 50°C getrocknet. Man erhielt 15 g (75 % d. Th.) der Verbindung der Formel

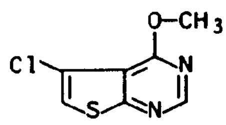

mit einem Schmelzpunkt von 145-146°C.

Beispiel 5

5 g 5-Chlor-4-thio-3,4-dihydro-thieno[2,3-d]pyrimidin wurden in 50 ml DMF und 5 ml Triethylamin gelöst. Hierzu wurden tropfenweise 5,4 g Bromethan gegeben. Das Reaktionsgemisch wurde 4 Stunden bei Raumtemperatur gerührt und anschließend in 500 ml Wasser eingerührt. Der Niederschlag wurde abgesaugt, mit Wasser gewaschen und im Vakuum bei 50°C getrocknet. Man erhielt 4,9 g (85 % d. Th.) der Verbindung der Formel

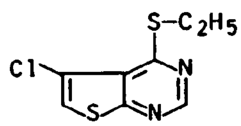

mit einem Schmelzpunkt von 64-66°C.

Beispiel 6

10,2 g 4,5-Dichlor-thieno[2,3-d]pyrimidin (Beispiel 1) wurden in 50 ml DMF und 4,5 g Pyridin gelöst. Hierzu wurden bei Raumtemperatur 4,5 g n-Butanthiol gegeben. Das Reaktionsgemisch wurde eine Stunde bei Raumtemperatur und weitere 2 Stunden bei 50-60°C gerührt. Anschließend wurden im Vakuum 10 ml Lösungsmittel abdestilliert. Der Rückstand wurde auf 500 ml Wasser gegeben, der Niederschlag abgesaugt, mit Wasser gewaschen und im Vakuum bei 50°C getrocknet. Man erhielt 9 g (70 % d. Th.) der Verbindung der Formel

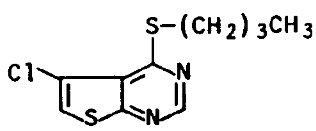

mit einem Schmelzpunkt von 118-120°C.

Beispiel 7

20,5 g 4,5-Dichlor-thieno[2,3-d]pyrimidin (Beispiel 1) wurden in 67 ml Dimethylformamid (DMF) gelöst. Hierzu wurden 12,4 g conc. Ammoniak, gelöst in 12 ml Wasser, getropft und das Reaktionsgemisch wurde 15 Stunden bei 50°C gerührt. Anschließend wurde das Gemisch auf 500 ml Wasser gegeben und der Niederschlag abgesaugt, mit Wasser gewaschen und im Vakuum bei 50°C getrocknet. Man erhielt 15,5 g (84 % d. Th.) der Verbindung der Formel

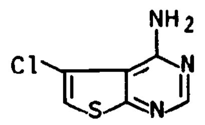

vom Schmelzpunkt 260-262°C.

Beispiel 8

Zu 50 ml einer 40 % wäßrigen Methylaminlösung in 20 ml Dimethylformamid wurden bei 20°C 15 g 4,5-Dichlor-thieno[2,3-d]pyrimidin (Beispiel 1) gegeben. Das Reaktionsgemisch wurde 4 Stunden bei 20°C gerührt. Anschließend wurde das Gemisch in 500 ml Wasser gegeben und der Niederschlag abgesaugt. Der Niederschlag wurde mit Wasser gewaschen und im Vakuum getrocknet. Man erhielt 11,5 g (79 % d. Th.) der Verbindung der Formel

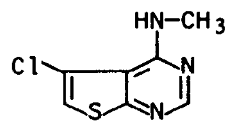

vom Schmelzpunkt 211-212°C.

Beispiel 9

12,1 g 4-Chlor-3-cyano-5-formyl-2-dimethylaminoformamidinothiophen und 50 ml Chlorbenzol wurden 3 Stunden bei Raumtemperatur sowie weitere 6 Stunden bei 100°C mit gasförmigem HCl begast. Anschließend wurde überschüssiges HCl mit Preßluft entfernt. Dem Reaktionsgemisch wurde dann 50 ml Wasser zugesetzt. Der sich daraufhin gebildete Niederschlag wurde abgesaugt, mit Wasser gewaschen und im Vakuum getrocknet. Es wurden 5,6 g (52 %) der Verbindung der Formel

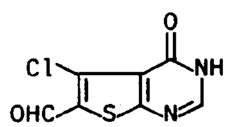

vom Schmelzpunkt 212°C (Zersetzung) erhalten.

Beispiel 10

33 g 2-Amino-3-carboxamido-4-oxo-4,5-dihydro-5-phenyl-thiophen wurden in 200 ml Ameisensäure 14 Stunden bei 100°C und weitere 16 Stunden unter Rückfluß gerührt. Nach dem Abkühlen wurde der Niederschlag abgesaugt, mit Wasser gewaschen und im Vakuum getrocknet. Es wurden 21,8 g (64 %) der Verbindung der Formel

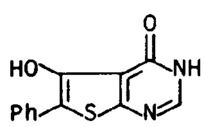

vom Schmelzpunkt über 290°C erhalten.

Beispiel 11

Variante a:

Zu einer Suspension von 14 g 2-Amino-3-cyano-4-oxo-4,5-dihydro-thiophen und 50 ml Phosphorylchlorid wurden portionsweise 19,6 g Benzoesäuremorpholid zugegeben. Anschließend wurde das Reaktionsgemisch eine Stunde bei Raumtemperatur und weitere drei Stunden bei 78°C gerührt. Dann wurde das Reaktionsgemisch in 500 ml Eiswasser gegossen. Der dabei angefallene Niederschlag wurde abfiltriert, mit Wasser gewaschen und im Vakuum getrocknet. Es wurden 23,3 g (83 %) der Verbindung der Formel

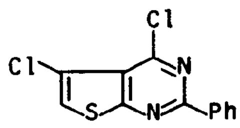

vom Schmelzpunkt 138 - 140°C erhalten.

Variante b:

Zu einer Suspension von 70 g 2-Amino-3-cyano-4-oxo-4,5-dihydro-thiophen und 250 ml Phosphorylchlorid wurden 88,5 g Benzoesäurediethylamid zugegeben. Danach wurde das Reaktionsgemisch 0,5 Stunden bei 20°C und 3 Stunden bei 78°C gerührt. Dann wurde das Reaktionsgemisch in 500 ml Eiswasser gegossen. Der dabei angefallene Niederschlag wurde abfiltriert, mit Wasser gewaschen und im Vakuum getrocknet. Dabei fielen 10,7 g (76 %) der unter Variante a erhaltenen Verbindung an.

In analoger Weise lassen sich die in Tabelle 1 aufgeführten Verbindungen herstellen.

Tabelle 1

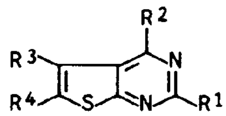

| Bsp. Nr. | Rest $R^1$ | Rest $R^2$ | Rest $R^3$ | Rest $R^4$ | Schmelzpunkt (°C) |
|---|---|---|---|---|---|
| 12 | H | Cl | Cl | $CH_3$ | 97- 99 |
| 13 | H | $O-C_2H_5$ | Cl | H | 124 |
| 14 | H | $O-nC_3H_7$ | Cl | H | 105-107 |
| 15 | H | $O-isoC_3H_7$ | Cl | H | 80- 81 |
| 16 | H | $O-nC_4H_9$ | Cl | H | 94- 95 |
| 17 | H | $O-isoC_4H_9$ | Cl | H | 79- 80 |
| 18 | H | $O-CH_2CH(OCH_3)CH_3$ | Cl | H | 66- 67 |
| 19 | H | $O-sekC_4H_9$ | Cl | H | öl |
| 20 | H | $O-nC_5H_{11}$ | Cl | H | 65- 66 |
| 21 | H | $O-NeoC_5H_{11}$ | Cl | H | |
| 22 | H | $O-nC_6H_{13}$ | Cl | H | öl |
| 23 | H | $O-nC_7H_{15}$ | Cl | H | öl |
| 24 | H | $O-nC_8H_{17}$ | Cl | H | öl |
| 25 | H | $O-isoC_8H_{17}$ | Cl | H | |
| 26 | H | $O-nC_9H_{19}$ | Cl | H | |
| 27 | H | $O-nC_{10}H_{21}$ | Cl | H | |
| 28 | H | $O-nC_{11}H_{23}$ | Cl | H | |
| 29 | H | $O-nC_{12}H_{25}$ | Cl | H | |
| 30 | H | $O-nC_{13}H_{27}$ | Cl | H | |
| 31 | H | $O-nC_{14}H_{31}$ | Cl | H | |
| 32 | H | $O-nC_{15}H_{31}$ | Cl | H | |
| 33 | H | $O-nC_{16}H_{31}$ | Cl | H | |
| 34 | H | $O-nC_{17}H_{33}$ | Cl | H | |
| 35 | H | $O-nC_{18}H_{35}$ | Cl | H | |
| 36 | H | $O-nC_{19}H_{37}$ | Cl | H | |
| 37 | H | $O-nC_{20}H_{39}$ | Cl | H | |
| 38 | H | $O-CH_2-CH=CH_2$ | Cl | H | 95- 98 |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 39 | H | $O-CH_2-C{\equiv}CH$ | Cl | H | 154-156 |
| 40 | H | O-Cyclopropyl | Cl | H | |
| 41 | H | O-Cyclobutyl | Cl | H | |
| 42 | H | O-Cyclopentyl | Cl | H | 85- 87 |
| 43 | H | O-Cyclohexyl | Cl | H | 90- 94 |
| 44 | H | O-Cycloheptyl | Cl | H | Öl |
| 45 | H | O-Cyclooctyl | Cl | H | |
| 46 | H | O-Cyclononyl | Cl | H | |
| 47 | H | O-Cyclodecyl | Cl | H | |
| 48 | H | O-Cyclododecyl | Cl | H | |
| 49 | H | O-Cyclohexyl-(2)-$CH_3$ | Cl | H | |
| 50 | H | $O-C_6H_5$ | Cl | H | 106-108 |
| 51 | H | $O-C_6H_4-(4)-CH_3$ | Cl | H | |
| 52 | H | $O-CH_2-C_6H_5$ | Cl | H | 99-100 |
| 53 | H | $O-CH_2-C_6H_4-(4)-CH_3$ | Cl | H | |
| 54 | H | $O-CH_2-C_6H_4-(4)-C(CH_3)_3$ | Cl | H | |
| 55 | H | $O-CH_2-CH_2-C_6H_5$ | Cl | H | 77- 78 |
| 56 | H | $O-CH_2CH_2-C_6H_4-(4)-OCH_3$ | Cl | H | |
| 57 | H | $O-CH_2CH_2OH$ | Cl | H | |
| 58 | H | $O-CH_2CH_2OCH_3$ | Cl | H | 107-109 |
| 59 | H | $O-CH_2CH_2OC_2H_5$ | Cl | H | 104-105 |
| 60 | H | $O-CH_2CH_2O-nC_3H_7$ | Cl | H | 60- 62 |
| 61 | H | $O-CH_2CH_2O-nC_4H_9$ | Cl | H | |
| 62 | H | $O-CH_2CH_2-NH-CH_2CH_2OH$ | Cl | H | 117-119 |
| 63 | H | $O-CH_2CH_2-N(CH_3)_2$ | Cl | H | 80- 84 |
| 64 | H | $O-CH_2CH_2-N(C_2H_5)_2$ | Cl | H | |
| 65 | H | $O-(CH_2)_3OH$ | Cl | H | |
| 66 | H | $O-(CH_2)_3OCH_3$ | Cl | H | |
| 67 | H | $O-(CH_2)_3OC_2H_5$ | Cl | H | |
| 68 | H | $O-CH_2CH_2O-nC_3H_7$ | Cl | H | |
| 69 | H | $O-CH_2CH_2O-nC_4H_9$ | Cl | H | |
| 70 | H | $O-(CH_2)_3-N(CH_3)_2$ | Cl | H | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 71 | H | $O-(CH_2)_3-N(C_2H_5)_2$ | Cl | H | |
| 72 | H | $O-CH(CH_3)-CH_2O-nC_4H_9$ | Cl | H | |
| 73 | H | $O-CH(C_2H_5)-CH_2OCH_3$ | Cl | H | |
| 74 | H | $O-(CH_2)_5-CH_2Cl$ | Cl | H | |
| 75 | H | $O-(CH_2)_7-CH_2Cl$ | Cl | H | |
| 76 | H | S-H | Cl | H | 264 |
| 77 | H | $S-CH_3$ | Cl | H | |
| 78 | H | $S-n-C_3H_7$ | Cl | H | 271 |
| 79 | H | $S-iso-C_3H_7$ | Cl | H | 260 |
| 80 | H | $S-iso-C_4H_9$ | Cl | H | 153-155 |
| 81 | H | $S-sek-C_4H_9$ | Cl | H | 148-150 |
| 82 | H | $S-tert-C_4H_9$ | Cl | H | 160-163 |
| 83 | H | $S-n-C_6H_{13}$ | Cl | H | |
| 84 | H | $S-n-C_8H_{17}$ | Cl | H | |
| 85 | H | $S-n-C_{12}H_{25}$ | Cl | H | |
| 86 | H | $S-n-C_{16}H_{33}$ | Cl | H | |
| 87 | H | $S-n-C_{18}H_{37}$ | Cl | H | |
| 88 | H | S-Cyclohexyl | Cl | H | |
| 89 | H | $S-C_6H_5$ | Cl | H | 181-182 |
| 90 | H | $S-C_6H_4-(4)-CH_3$ | Cl | H | |
| 91 | H | $S-C_6H_4-(2)-OH$ | Cl | H | |
| 92 | H | $S-C_6H_4-(4)-OCH_3$ | Cl | H | |
| 93 | H | $S-C_6H_4-(4)-Cl$ | Cl | H | |
| 94 | H | $S-C_6H_3-(2,6)-Cl_2$ | Cl | H | |
| 95 | H | $S-CH_2-C_6H_5$ | Cl | H | |
| 96 | H | $S-(CH_2)_2-OH$ | Cl | H | |
| 97 | H | $S-(CH_2)_2-N(C_2H_5)_2$ | Cl | H | |
| 98 | H | $S-CH_2CH(CH_3)-OH$ | Cl | H | |
| 99 | H | $S-(CH_2)_2-COOH$ | Cl | H | |
| 100 | H | $S-CH(CH_3)-COOH$ | Cl | H | |
| 101 | H | $S-CH_2-COOH$ | Cl | H | |
| 102 | H | $S-CH_2-COOCH_3$ | Cl | H | 105-108 |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 103 | H | $S-CH_2-COOC_2H_5$ | Cl | H | 105-107 |
| 104 | H | $S-CH_2$-(2)-Furyl | Cl | H | |
| 105 | H | SCN | Cl | H | 208-210 |
| 106 | H | F | Cl | H | |
| 107 | H | CN | Cl | H | |
| 108 | H | $N_3$ | Cl | H | 208 Zers. |
| 109 | H | $NH-(CH_2)_4-CH_3$ | Cl | $CH_3$ | Öl |
| 110 | H | $O-CH_3$ | Cl | $CH_3$ | 92- 93 |
| 111 | H | $O-C_2H_5$ | Cl | $CH_3$ | 74- 75 |
| 112 | H | $O-C_6H_5$ | Cl | $CH_3$ | 108-109 |
| 113 | H | S-H | Cl | $CH_3$ | 241-243 |
| 114 | H | $S-C_6H_5$ | Cl | $CH_3$ | 131-133 |
| 115 | H | $S-CH_2-COOC_2H_5$ | Cl | $CH_3$ | 94- 98 |
| 116 | H | Cl | Cl | $CHCl_2$ | 125-130 |
| 117 | H | Cl | Cl | CN | 155-157 |
| 118 | H | $CH_2COOC_2H_5$ | Cl | H | 91- 92 |
| 119 | H | $CH(COOC_2H_5)_2$ | Cl | H | 112-113 |
| 120 | H | $CH(CN)_2$ | Cl | H | |
| 121 | H | $CH(CN)COOC_2H_5$ | Cl | H | 158-159 |
| 122 | H | $CH(CN)COOCH_3$ | Cl | H | |
| 123 | H | $CH(COOCH_3)_2$ | Cl | H | |
| 124 | H | $CH(CN)C_6H_5$ | Cl | H | |
| 125 | H | $NH-NH-C_2H_5$ | Cl | H | |
| 126 | H | $NH-N(C_2H_5)_2$ | Cl | H | |
| 127 | H | $S-n-C_5H_{11}$ | Cl | H | |
| 128 | H | $S-Neo-C_5H_{11}$ | Cl | H | |
| 129 | H | $N(CH_3)-NH-CH_3$ | Cl | H | |
| 130 | H | OH | OH | H | |
| 131 | $C_2H_5$ | OH | OH | H | |
| 132 | $C_2H_5$ | Cl | Cl | H | |
| 133 | $nC_3H_7$ | OH | OH | H | |
| 134 | $nC_3H_7$ | Cl | Cl | H | |
| 135 | $isoC_3H_7$ | OH | OH | H | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 136 | iso$C_3H_7$ | Cl | Cl | H | |
| 137 | n$C_4H_9$ | OH | OH | H | |
| 138 | n$C_4H_9$ | Cl | Cl | H | |
| 139 | Cyclo$C_3H_5$ | OH | OH | H | |
| 140 | Cyclo$C_3H_5$ | Cl | Cl | H | |
| 141 | Cyclo$C_6H_{11}$ | OH | OH | H | |
| 142 | Cyclo$C_6H_{11}$ | Cl | Cl | H | |
| 143 | H | NH-$C_2H_5$ | Cl | H | 134-135 |
| 144 | H | NH-n$C_3H_7$ | Cl | H | 71- 75 |
| 145 | H | NH-iso$C_3H_7$ | Cl | H | 119-120 |
| 146 | H | NH-n$C_4H_9$ | Cl | H | 71- 75 |
| 147 | H | NH-sek$C_4H_9$ | Cl | H | 54- 58 |
| 148 | H | NH-iso$C_4H_9$ | Cl | H | 88- 90 |
| 149 | H | NH-tert$C_4H_9$ | Cl | H | 80- 81 |
| 150 | H | NH-n$C_5H_{11}$ | Cl | H | 76- 77 |
| 151 | H | NH-sek$C_5H_{11}$ | Cl | H | öl |
| 152 | H | NH-$CH(CH_3)-CH(CH_3)_2$ | Cl | H | |
| 153 | H | NH-Neo$C_5H_{11}$ | Cl | H | 111-114 |
| 154 | H | NH-n$C_6H_{13}$ | Cl | H | 50 |
| 155 | H | NH-n$C_7H_{15}$ | Cl | H | 64 |
| 156 | H | NH-n$C_8H_{17}$ | Cl | H | 54- 56 |
| 157 | H | NH-n$C_9H_{19}$ | Cl | H | 60- 65 |
| 158 | H | NH-n$C_{10}H_{21}$ | Cl | H | |
| 159 | H | NH-n$C_{12}H_{25}$ | Cl | H | |
| 160 | H | NH-n$C_{13}H_{27}$ | Cl | H | |
| 161 | H | NH-n$C_{16}H_{33}$ | Cl | H | |
| 162 | H | NH-n$C_{18}H_{37}$ | Cl | H | |
| 163 | H | NH-$CH(CH_3)CH_2CH_2CH_3$ | Cl | H | öl |
| 164 | H | NH-$CH(CH_3)-(CH_2)_3CH_3$ | Cl | H | öl |
| 165 | H | NH-$CH(CH_3)-(CH_2)_2-CH(CH_3)_2$ | Cl | H | öl |
| 166 | H | NH-$CH(CH_3)-(CH_2)_5-CH_3$ | Cl | H | |
| 167 | H | NH-$CH(C_2H_5)-(CH_2)_4-CH_3$ | Cl | H | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 168 | H | $NH-CH(CH_3)-(CH_2)_3-CH(CH_3)_2$ | Cl | H | öl |
| 169 | H | (R)-(-)-NH-CH($CH_3$)-Cyclohexyl | Cl | H | |
| 170 | H | (S)-(+)-NH-CH($CH_3$)-Cyclohexyl | Cl | H | |
| 171 | H | $NH-CH_2CH_2$-Cyclohexyl | Cl | H | 69- 71 |
| 172 | H | $NH-(CH_2)_2-OH$ | Cl | H | 143-145 |
| 173 | H | $NH-(CH_2)_2-OCH_3$ | Cl | H | 115-117 |
| 174 | H | $NH-(CH_2)_2-OC_2H_5$ | Cl | H | 80- 83 |
| 175 | H | $NH-(CH_2)_2-OC_6H_5$ | Cl | H | |
| 176 | H | $NH-(CH_2)_2-O-(CH_2)_2-OH$ | Cl | H | |
| 177 | H | $NH-(CH_2)_2-NH-(CH_2)_2-OH$ | Cl | H | |
| 178 | H | $NH-(CH_2)_2-NH_2$ | Cl | H | |
| 179 | H | $NH-(CH_2)_2-N(CH_3)_2$ | Cl | H | 146-148 |
| 180 | H | $NH-(CH_2)_2-N(C_2H_5)_2$ | Cl | H | 67- 70 |
| 181 | H | $NH-(CH_2)_2-N(iso-C_3H_7)_2$ | Cl | H | |
| 182 | H | $NH-(CH_2)_2$-Piperazin-(1)-yl | Cl | H | |
| 183 | H | $NH-(CH_2)_3-OH$ | Cl | H | 114-116 |
| 184 | H | $NH-(CH_2)_3-OCH_3$ | Cl | H | |
| 185 | H | $NH-(CH_2)_3-OC_2H_5$ | Cl | H | 80- 83 |
| 186 | H | $NH-(CH_2)_3-OCH(CH_3)_2$ | Cl | H | 42- 43 |
| 187 | H | $NH-(CH_2)_3-OC_6H_5$ | Cl | H | |
| 188 | H | $NH-(CH_2)_3-O-CH_2C_6H_5$ | Cl | H | öl |
| 189 | H | $NH-(CH_2)_3-O-(CH_2)_2-O-CH_3$ | Cl | H | |
| 190 | H | $NH-(CH_2)_3-O-(CH_2)_2-O-C_2H_5$ | Cl | H | |
| 191 | H | $NH-(CH_2)_3-O-(CH_2)_2-O-C_6H_5$ | Cl | H | öl |
| 192 | H | $NH-(CH_2)_3-NH_2$ | Cl | H | 228-230 |
| 193 | H | $NH-(CH_2)_3-N(CH_3)_2$ | Cl | H | 112-115 |
| 194 | H | $NH-(CH_2)_3-N(C_2H_5)_2$ | Cl | H | 66- 68 |
| 195 | H | $NH-(CH_2)_3-N(n-C_4H_9)_2$ | Cl | H | |
| 196 | H | $NH-(CH_2)_3$-Morpholin-(1)-yl | Cl | H | |
| 197 | H | $NH-(CH_2)_4-OH$ | Cl | H | |
| 198 | H | $NH-(CH_2)_5-NH_2$ | Cl | H | |
| 199 | H | $NH-(CH_2)_6-OH$ | Cl | H | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 200 | H | $NH-(CH_2)_6-NH_2$ | Cl | H | |
| 201 | H | $NH-(CH_2)_7-NH_2$ | Cl | H | |
| 202 | H | $NH-(CH_2)_8-NH_2$ | Cl | H | |
| 203 | H | $NH-(CH_2)_9-NH_2$ | Cl | H | |
| 204 | H | $NH-(CH_2)_{10}-NH_2$ | Cl | H | |
| 205 | H | $NH-(CH_2)_{12}-NH_2$ | Cl | H | |
| 206 | H | $NH-CH(CH_3)-(CH_2)_3-N(C_2H)_2$ x HCl | Cl | H | 165-170 |
| 207 | H | $NH-CH_2-CH(CH_3)-OH$ | Cl | H | |
| 208 | H | $NH-CH(CH_3)-CH_2-OH$ | Cl | H | |
| 209 | H | $NH-CH_2$-(2)-Thienyl | Cl | H | |
| 210 | H | $NH-C(CH_3)_2-CH_2-OH$ | Cl | H | |
| 211 | H | $NH-C(CH_3)-(CH_2-OH)_2$ | Cl | H | |
| 212 | H | $NH-CH(C_2H_5)-CH_2-OH$ | Cl | H | |
| 213 | H | $NH-(CH_2)_2-COOH$ | Cl | H | |
| 214 | H | $NH-(CH_2)_5-COOH$ | Cl | H | |
| 215 | H | $NH-(CH_2)_{10}-COOH$ | Cl | H | |
| 216 | H | $NH-C_6H_5$ | Cl | H | 86- 88 |
| 217 | H | $NH-C_6H_4-(2)-CH_3$ | Cl | H | 126-128 |
| 218 | H | $NH-C_6H_4-(3)-CH_3$ | Cl | H | 116-118 |
| 219 | H | $NH-C_6H_4-(4)-CH_3$ | Cl | H | 162-165 |
| 220 | H | $NH-C_6H_4-(2)-C_2H_5$ | Cl | H | 94- 96 |
| 221 | H | $NH-C_6H_4-(4)-C_2H_5$ | Cl | H | 71- 73 |
| 222 | H | $NH-C_6H_4-(2)-CF_3$ | Cl | H | |
| 223 | H | $NH-C_6H_4-(3)-CF_3$ | Cl | H | 98-100 |
| 224 | H | $NH-C_6H_4-(4)-CF_3$ | Cl | H | |
| 225 | H | $NH-C_6H_5-(2)-OH$ | Cl | H | 218-220 |
| 226 | H | $NH-C_6H_5-(3)-OH$ | Cl | H | 238-241 |
| 227 | H | $NH-C_6H_5-(4)-OH$ | Cl | H | 219-222 |
| 228 | H | $NH-C_6H_4-(2)-OCH_3$ | Cl | H | 167-169 |
| 229 | H | $NH-C_6H_4-(3)-OCH_3$ | Cl | H | 114-115 |
| 230 | H | $NH-C_6H_4-(4)-OCH_3$ | Cl | H | 104-107 |
| 231 | H | $NH-C_6H_4-(2)-OC_2H_5$ | Cl | H | 152-153 |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 232 | H | $NH-C_6H_4-(3)-OC_2H_5$ | Cl | H | 93- 95 |
| 233 | H | $NH-C_6H_4-(4)-OC_2H_5$ | Cl | H | 128-130 |
| 234 | H | $NH-C_6H_4-(2)-Cl$ | Cl | H | 126-130 |
| 235 | H | $NH-C_6H_4-(3)-Cl$ | Cl | H | 135-137 |
| 236 | H | $NH-C_6H_4-(4)-Cl$ | Cl | H | 191-195 |
| 237 | H | $NH-C_6H_4-(2)-F$ | Cl | H | |
| 238 | H | $NH-C_6H_4-(3)-F$ | Cl | H | |
| 239 | H | $NH-C_6H_4-(4)-F$ | Cl | H | |
| 240 | H | $NH-C_6H_4-(2)-Br$ | Cl | H | |
| 241 | H | $NH-C_6H_4-(3)-Br$ | Cl | H | |
| 242 | H | $NH-C_6H_4-(4)-Br$ | Cl | H | |
| 243 | H | $NH-C_6H_4-(2)-I$ | Cl | H | |
| 244 | H | $NH-C_6H_4-(3)-I$ | Cl | H | |
| 245 | H | $NH-C_6H_4-(4)-I$ | Cl | H | |
| 246 | H | $NH-C_6H_4-(2)-NH_2$ | Cl | H | 154-156 |
| 247 | H | $NH-C_6H_4-(3)-NH_2$ | Cl | H | 182-185 |
| 248 | H | $NH-C_6H_4-(4)-NH_2$ | Cl | H | 184-185 |
| 249 | H | $NH-C_6H_4-(4)-NH-COCH_3$ | Cl | H | 210-212 |
| 250 | H | $NH-C_6H_4-(2)-CH_2OH$ | Cl | H | |
| 251 | H | $NH-C_6H_4-(3)-CH_2OH$ | Cl | H | |
| 252 | H | $NH-C_6H_4-(4)-COCH_3$ | Cl | H | 194-195 |
| 253 | H | $NH-C_6H_4-(2)-COOH$ | Cl | H | |
| 254 | H | $NH-C_6H_4-(3)-COOH$ | Cl | H | |
| 255 | H | $NH-C_6H_4-(4)-COOH$ | Cl | H | |
| 256 | H | $NH-C_6H_4-(2)-CONH_2$ | Cl | H | |
| 257 | H | $NH-C_6H_4-(4)-CONH_2$ | Cl | H | |
| 258 | H | $NH-C_6H_4-(2)-CN$ | Cl | H | |
| 259 | H | $NH-C_6H_4-(3)-CN$ | Cl | H | |
| 260 | H | $NH-C_6H_4-(4)-CN$ | Cl | H | 210-213 |
| 261 | H | $NH-C_6H_3-(2,6)-(CH_3)_2$ | Cl | H | |
| 262 | H | $NH-C_6H_3-(3,5)-(CH_3)_2$ | Cl | H | |
| 263 | H | $NH-C_6H_3-(2)-OH-(5)-CH_3$ | Cl | H | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 264 | H | $NH-C_6H_3-(2)-OH-(4)-CH_3$ | Cl | H | 258 |
| 265 | H | $NH-C_6H_3-(2)-OH-(2)-CH_3$ | Cl | H | |
| 266 | H | $NH-C_6H_3-(2)-OH-(5)-Cl$ | Cl | H | |
| 267 | H | NH-(2)-Pyridyl | Cl | H | 175- 6 |
| 268 | H | NH-(3)-Pyridyl | Cl | H | 138-140 |
| 269 | H | NH-(4)-Pyridyl | Cl | H | |
| 270 | H | NH-(2)-(4'-Methyl-pyridyl) | Cl | H | |
| 271 | H | NH-(2)-(5'-Methyl-pyridyl) | Cl | H | |
| 272 | H | NH-(2)-(6'-Methyl-pyridyl) | Cl | H | |
| 273 | H | $NH-CH_2-(3)$-Pyridyl | Cl | H | |
| 274 | H | $NH-CH_2-(2)$-Furyl | Cl | H | |
| 275 | H | $NH-CH_2-C_6H_5$ | Cl | H | 98- 99 |
| 276 | H | $NH-CH_2-C_6H_4-(2)-OCH_3$ | Cl | H | |
| 277 | H | $NH-CH_2-C_6H_4-(4)-OCH_3$ | Cl | H | 118-120 |
| 278 | H | $NH-CH_2-C_6H_4-(4)-F$ | Cl | H | |
| 279 | H | $NH-CH_2-C_6H_4-(2)-Cl$ | Cl | H | |
| 280 | H | $NH-CH_2-C_6H_4-(4)-Cl$ | Cl | H | |
| 281 | H | $NH-CH_2-C_6H_3-(3,4)-(OCH_3)_2$ | Cl | H | |
| 282 | H | $NH-CH_2-CH_2-C_6H_5$ | Cl | H | 83 |
| 283 | H | $NH-CH(CH_3)-C_6H_5$ (+/-) | Cl | H | 76- 78 |
| 284 | H | $NH-(CH_2)_3-C_6H_5$ | Cl | H | |
| 285 | H | $NH-CH(CH_3)-(CH_2)_2-C_6H_5$ | Cl | H | |
| 286 | H | NH-Cyclopropyl | Cl | H | 87 |
| 287 | H | NH-Cyclopentyl | Cl | H | 79- 80 |
| 288 | H | NH-Cyclohexyl | Cl | H | 85- 86 |
| 289 | H | NH-Cycloheptyl | Cl | H | 62- 63 |
| 290 | H | NH-Cyclooctyl | Cl | H | 43- 48 |
| 291 | H | NH-Cyclododecyl | Cl | H | 119-122 |
| 292 | H | $NH-CH_2-CH=CH_2$ | Cl | H | 85- 86 |
| 293 | H | $NH-CH_2-C\equiv CH$ | Cl | H | 171-173 |
| 294 | H | $N(CH_3)_2$ | Cl | H | 108-110 |
| 295 | H | $N(C_2H_5)_2$ | Cl | H | 42- 45 |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 296 | H | $N(n-C_3H_7)_2$ | Cl | H | Öl |
| 297 | H | $N(n-C_4H_9)2$ | Cl | H | Öl |
| 298 | H | $N(iso-C_4H_9)_2$ | Cl | H | 69- 72 |
| 299 | H | $N(n-C_5H_{11})_2$ | Cl | H | Öl |
| 300 | H | $N(sek-C_4H_9)_2$ | Cl | H | |
| 301 | H | $N(iso-C_5H_{11})_2$ | Cl | H | |
| 302 | H | $N(n-C_6H_{13})_2$ | Cl | H | Öl |
| 303 | H | $N(CH(C_2H_5)-(CH_2)_4-CH_3)_2$ | Cl | H | |
| 304 | H | $N(n-C_8H_{17})_2$ | Cl | H | |
| 305 | H | $N(CH_2-CH=CH_2)_2$ | Cl | H | Öl |
| 306 | H | $N(Cyclohexyl)_2$ | Cl | H | Öl |
| 307 | H | $N(CH_3)-nC_4H_9$ | Cl | H | |
| 308 | H | $N(CH_3)$-Cyclohexyl | Cl | H | |
| 309 | H | $N(C_2H_5)$-Cyclohexyl | Cl | H | Öl |
| 310 | H | $N(CH_3)-(CH_2)_2-OH$ | Cl | H | |
| 311 | H | $N(C_2H_5)-CH(CH_3)-CH(CH_3)_2$ | Cl | H | |
| 312 | H | $N(C_2H_5)-(CH)_2-OH$ | Cl | H | |
| 313 | H | $N(C_2H_5)-CH_2-CH(OH)-CH_3$ | Cl | H | |
| 314 | H | $N(tert-C_4H_9)-(CH_2)_2-OH$ | Cl | H | |
| 315 | H | $N(CH_3)C_6H_5$ | Cl | H | 165-167 |
| 316 | H | $N(C_2H_5)-C_6H_5$ | Cl | H | |
| 317 | H | $N(iso-C_3H_7)-C_6H_5$ | Cl | H | |
| 318 | H | $N(nC_4H_9)-C_6H_5$ | Cl | H | |
| 319 | H | $N(CH_2C_6H_5)_2$ | Cl | H | 101-102 |
| 320 | H | $N(CH_3)-CH_2C_6H_5$ | Cl | H | |
| 321 | H | $N(C_2H_5)-CH_2C_6H_5$ | Cl | H | |
| 322 | H | $N(iso-C_3H_7)-CH_2C_6H_5$ | Cl | H | |
| 323 | H | $N(tert-C_4H_9)-CH_2C_6H_5$ | Cl | H | |
| 324 | H | $N(CH_2C_6H_5)-(CH_2)_2-OH$ | Cl | H | |
| 325 | H | $N(C_6H_5)-CH_2C_6H_5$ | Cl | H | |
| 326 | H | $N(CH_2C_6H_5)-((CH_2)_2-C_6H_5)$ | Cl | H | |
| 327 | H | Pyrrolidino | Cl | H | 110-112 |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 328 | H | Piperidino | Cl | H | 102-105 |
| 329 | H | Hexamethylenimino | Cl | H | 255-256 |
| 330 | H | Morpholino | Cl | H | 162-165 |
| 331 | H | N-Methylpiperazino | Cl | H | 116-120 |
| 332 | H | $NH-NH_2$ | Cl | H | 229-230 |
| 333 | H | $NH-NH-CH_3$ | Cl | H | |
| 334 | H | $NH-NH-C_6H_5$ | Cl | H | 134-136 |
| 335 | H | $NH-N(CH_3)_2$ | Cl | H | |
| 336 | H | NH-Morpholin-(1)-yl | Cl | H | |
| 337 | H | NH-Hexamethylenimino-(1)-yl | Cl | H | |
| 338 | H | NH-OH | Cl | H | |
| 339 | $CH_3$ | $NH-CH_3$ | Cl | H | 148-150 |
| 340 | $CH_3$ | $NH-C_2H_5$ | Cl | H | 208-210 |
| 341 | $CH_3$ | $NH-nC_3H_7$ | Cl | H | 150-151 |
| 342 | $CH_3$ | $NH-isoC_3H_7$ | Cl | H | |
| 343 | $CH_3$ | $NH-sekC_4H_9$ | Cl | H | 187-190 |
| 344 | $CH_3$ | $NH-nC_5H_{11}$ | Cl | H | 60- 62 |
| 345 | $CH_3$ | NH-Cyclohexyl | Cl | H | |
| 346 | $CH_3$ | $NH-C_6H_5$ | Cl | H | 109-112 |
| 347 | $CH_3$ | $N(C_2H_5)_2$ | Cl | H | 208-210 |
| 348 | $CH_2Cl$ | $NH-CH_3$ | Cl | H | |
| 349 | $CH_2Cl$ | $NH-C_2H_5$ | Cl | H | 93- 97 |
| 350 | H | $NH-CH_3$ | Cl | $CH_3$ | 186-187 |
| 351*) | H | $NH-C_2H_5$ | Cl | $CH_3$ | 128-130 |
| 352*) | H | $NH-nC_3H_7$ | Cl | $CH_3$ | 61- 65 |
| 353*) | H | $NH-nC_4H_9$ | Cl | $CH_3$ | 61- 65 |
| 354 | H | $NH-sekC_4H_9$ | Cl | $CH_3$ | 101-102 |
| 355 | H | $NH-nC_6H_{13}$ | Cl | $CH_3$ | 43- 44 |
| 356 | H | $NH-nC_7H_{15}$ | Cl | $CH_3$ | 53- 54 |
| 357 | H | $NH-C_2H_4-OH$ | Cl | $CH_3$ | 155-159 |
| 358 | H | $NH-C_2H_4-OCH_3$ | Cl | $CH_3$ | 87- 88 |
| 359 | H | NH-Cyclohexyl | Cl | $CH_3$ | 111-112 |

*) HCl-Salz

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 360 | H | $NH-C_6H_5$ | Cl | $CH_3$ | 169-171 |
| 361 | H | $NH-C_6H_4-(3)-CH_3$ | Cl | $CH_3$ | 152-154 |
| 362 | H | $NH-CH_2-C{\equiv}CH$ | Cl | $CH_3$ | 183-186 |
| 363 | H | $N(CH_3)_2$ | Cl | $CH_3$ | 60- 64 |
| 364 | H | Pyrrolidino | Cl | $CH_3$ | 75- 76 |
| 365 | H | Piperidino | Cl | $CH_3$ | 128-130 |
| 366 | H | Morpholino | Cl | $CH_3$ | 105-106 |
| 367 | H | $NH-NH_2$ | Cl | $CH_3$ | 194-195 |
| 368 | H | $NH-nC_3H_7$ | Cl | CN | 99-102 |
| 369 | H | $NH-nC_4H_9$ | Cl | CN | 90- 92 |
| 370 | H | $NH-sekC_4H_9$ | Cl | CN | 82- 84 |
| 371 | H | $NH-nC_5H_{11}$ | Cl | CN | 82- 84 |
| 372 | H | $N(C_2H_5)_2$ | Cl | CN | 60- 62 |
| 373 | H | $NH-CH_3$ | Cl | $CH=N-CH_3$ | 206-208 |
| 374 | H | $NH-isoC_3H_7$ | Cl | $CH=N-isoC_3H_7$ | 105-112 |
| 375 | H | $(R/S)-NH-CH(CH_3)-$Cyclohexyl | Cl | H | |
| 376 | H | $NH-(CH_2)_3-N(n-C_3H_7)_2$ | Cl | H | |
| 377 | H | $NH-(CH_2)_3-N(isoC_3H_7)_2$ | Cl | H | |
| 378 | H | $NH-(+)-CH(CH_3)-C_6H_5$ | Cl | H | |
| 379 | H | $NH-(-)-CH(CH_3)-C_6H_5$ | Cl | H | |
| 380 | H | NH-Cyclobutyl | Cl | H | |
| 381 | H | NH-Adamantyl | Cl | H | |
| 382 | H | NH-Cyclononyl | Cl | H | |
| 383 | H | NH-Cyclodecyl | Cl | H | |
| 384 | H | NH-Metallyl | Cl | H | |
| 385 | H | $N(IsoC_3H_7)_2$ | Cl | H | |
| 386 | H | N-Ethylpiperazino | Cl | H | |
| 387 | H | Thiomorpholino | Cl | H | |
| 388 | H | $NH-NH-C_2H_5$ | Cl | H | |
| 389 | H | $NH-N(C_2H_5)_2$ | Cl | H | |
| 390 | H | $N(CH_3)-NH-CH_3$ | Cl | H | |
| 391 | H | Imidazol-1-yl | Cl | H | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 392 | H | Pyrazol-1-yl | Cl | H | |
| 393 | H | Triazol-1-yl | Cl | H | |
| 394 | H | Tetrazol-1-yl | Cl | H | |
| 395 | H | $NH-CH(CH_3)-(CH_2)_4CH_3$ | Cl | H | Öl |
| 396 | H | $NH-CH(CH_3)-CH_2CN$ | Cl | H | 123-125 |
| 397 | H | $N(CH_3)-CH(CH_3)-CH_2CN$ | Cl | H | 84- 90 |
| 398 | H | $NH-CH(CH_3)-(CH_2)_4CH_3$ | Cl | H | Öl |
| 399 | H | $N(C_2H_5)_2$ | Cl | $CH_3$ | Öl |
| 400 | $CH_3$ | $NH-(CH_2)_3-OH$ | Cl | H | 150-152 |
| 401 | H | $NH-(CH_2)_3-COOH$ | Cl | H | 187-190 |
| 402 | H | $NH-nC_6H_{13}$ | Cl | CN | 64- 65 |
| 403 | H | SCN | Cl | $CH_3$ | 162-165 |
| 404 | H | $NH-C_6H_5$ | Cl | $CH=N-C_6H_5$ | 180-183 |
| 405 | H | $NH-(CH_2)_2$-Cyclohexyl | Cl | $CH_3$ | 73- 74 |
| 406 | H | $NH-nC_7H_{15}$ | Cl | CN | 64- 65 |
| 407 | H | $NH-nC_3H_7$ | Cl | $CH=N-nC_3H_7$ | 77- 83 |
| 408 | H | $NH-CH_2CH_2C_6H_5$ | Cl | $CH_3$ | 82- 84 |
| 409 | H | $NH-nC_8H_{17}$ | Cl | $CH_3$ | 41- 43 |
| 410 | H | $NH-CH(CH_3)-C_6H_5$ | Cl | $CH_3$ | 121-123 |
| 411 | H | $NH-CH(CH_3)-(CH_2)_4-CH_3$ | Cl | $CH_3$ | Öl |
| 412 | H | $NH-CH(CH_3)-(CH_2)_3-CH(CH_3)_2$ | Cl | $CH_3$ | Öl |
| 413 | H | $NH-(CH_2)_3-OCH_3$ | Cl | $CH_3$ | 71- 72 |
| 414 | H | $NH-CH_2-C_6H_5$ | Cl | $CH_3$ | 133-134 |
| 415 | H | $NH-nC_9H_{19}$ | Cl | $CH_3$ | Öl |
| 416 | H | $NH-C_6H_2-(4)-NH_2-(3,5)-Cl_2$ | Cl | H | 242 |
| 417 | H | $NH-(CH_2)_3-O-(CH_2)_2O(CH_2)_2OC_2H_5$ | Cl | H | Öl |
| 418 | H | $NH-CH(CH_3)-(CH_2)_2CH(CH_3)_2$ | Cl | $CH_3$ | Öl |
| 419 | H | $O-CH(CH_3)-CH_2OCH_3$ | Cl | H | Öl |
| 420 | H | $O-CH(CH_3)-CH_2OC_6H_5$ | Cl | H | 80- 82 |
| 421 | H | $S-CH_2C\equiv CH$ | Cl | H | 170-172 |
| 422 | H | $S-CH_2CH=CH_2$ | Cl | H | 58- 62 |
| 423 | H | $NH-C_6H_4-(4)-COOC_2H_5$ | Cl | H | 158-162 |

Tabelle 1 (Fortsetzung)

| Bsp. Nr. | Rest R1 | Rest R2 | Rest R3 | Rest R4 | Schmelzpunkt (°C) |
|---|---|---|---|---|---|
| 424 | H | $NH-nC_8H_{17}$ | Cl | CN | 45-46 |
| 425 | H | $NH-CH_2-CH(C_2H_5)-(CH_2)_3CH_3$ | Cl | H | Öl |
| 426 | H | $NH-(CH_2)_3OC_2H_5$ | Cl | CN | 70-74 |
| 427 | H | $N(CH_2CH_2OCH_3)_2$ | Cl | H | Öl |
| 428 | H | $S-nC_5H_{11}$ | Cl | H | 170-172 |
| 429 | H | $S-CH(CH_2CH_2CH_3)-(CH_2)_3CH_3$ | Cl | H | Öl |
| 430 | H | $NH-(CH_2)_8-CH=CH-(CH_2)_7CH_3$ | Cl | H | Öl |
| 431 | $CH_3$ | Cl | Cl | H | 126-130 |
| 432 | $CH_2Cl$ | Cl | Cl | H | 120-124 |
| 433 | H | Thiomorpholino-S,S-dioxid | Cl | H | über 300 |
| 434 | H | $NH-CH_3$ | Cl | CHO | |
| 435 | H | $NH-C_2H_5$ | Cl | CHO | |
| 436 | H | $NH-nC_3H_7$ | Cl | CHO | |
| 437 | H | $NH-isoC_3H_7$ | Cl | CHO | |
| 438 | H | $NH-nC_4H_9$ | Cl | CHO | |
| 439 | H | $NH-isoC_4H_9$ | Cl | CHO | |
| 440 | H | $NH-secC_4H_9$ | Cl | CHO | |
| 441 | H | $NH-tertC_4H_9$ | Cl | CHO | |
| 442 | H | $NH-nC_5H_{11}$ | Cl | CHO | |
| 443 | H | $NH-NeoC_5H_{11}$ | Cl | CHO | |
| 444 | H | $NH-nC_6H_{13}$ | Cl | CHO | 85-87 |
| 445 | H | $NH-nC_7H_{15}$ | Cl | CHO | |
| 446 | H | $NH-nC_8H_{17}$ | Cl | CHO | 49-54 |
| 447 | H | $NH-nC_9H_{19}$ | Cl | CHO | |
| 448 | H | $NH-nC_{10}H_{21}$ | Cl | CHO | Öl |
| 449 | H | $NH-nC_{11}H_{23}$ | Cl | CHO | Öl |
| 450 | H | $NH-nC_{12}H_{25}$ | Cl | CHO | Öl |
| 451 | H | $NH-nC_{13}H_{27}$ | Cl | CHO | Öl |
| 452 | H | $NH-nC_{14}H_{29}$ | Cl | CHO | Öl |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 453 | H | $NH-nC_{15}H_{31}$ | Cl | CHO | Öl |
| 454 | H | $NH-nC_{16}H_{33}$ | Cl | CHO | Öl |
| 455 | H | $NH-nC_{17}H_{35}$ | Cl | CHO | Öl |
| 456 | H | $NH-nC_{18}H_{37}$ | Cl | CHO | Öl |
| 457 | H | $NH-nC_{19}H_{39}$ | Cl | CHO | Öl |
| 458 | H | $NH-nC_{20}H_{41}$ | Cl | CHO | Öl |
| 459 | H | $NH-CH(CH_3)CH_2CH_2CH_3$ | Cl | CHO | Öl |
| 460 | H | $NH-CH(CH_3)-(CH_2)_3CH_3$ | Cl | CHO | Öl |
| 461 | H | $NH-C_6H_5$ | Cl | CHO | |
| 462 | H | $NH-CH_2C_6H_5$ | Cl | CHO | |
| 463 | H | $NH-CH_2CH_2C_6H_5$ | Cl | CHO | |
| 464 | H | $NH-CH(CH_3)-C_6H_5$ | Cl | CHO | |
| 465 | H | $NH-(CH_2)_3-C_6H_5$ | Cl | CHO | |
| 466 | H | $NH-CH_2-COOH$ | Cl | CHO | |
| 467 | H | $NH-(CH_2)_2-COOH$ | Cl | CHO | |
| 468 | H | $NH-(CH_2)_3-COOH$ | Cl | CHO | |
| 469 | H | $NH-CH(CH_3)-(CH_2)_3-CH(CH_3)_2$ | Cl | CHO | Öl |
| 470 | H | $NH-(CH_2)_2-OH$ | Cl | CHO | |
| 471 | H | $NH-(CH_2)_3-OH$ | Cl | CHO | |
| 472 | $C_6H_5$ | Thiomorpholino-S,S-dioxid | Cl | H | |
| 473 | H | $NH-(CH_2)_2-OCH_3$ | Cl | CHO | |
| 474 | H | $NH-(CH_2)_2-OC_2H_5$ | Cl | CHO | |
| 475 | H | $NH-(CH_2)_3-OCH_3$ | Cl | CHO | |
| 476 | H | $NH-(CH_2)_3-OC_2H_5$ | Cl | CHO | |
| 477 | H | NH-Cyclopropyl | Cl | CHO | |
| 478 | H | NH-Cyclopentyl | Cl | CHO | |
| 479 | H | NH-Cyclohexyl | Cl | CHO | |
| 480 | H | NH-Cycloheptyl | Cl | CHO | |
| 481 | H | NH-Cyclooctyl | Cl | CHO | |
| 482 | H | NH-Cyclododecyl | Cl | CHO | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 483 | $C_6H_5$ | $NH-CH_3$ | Cl | H | |
| 484 | $C_6H_5$ | $NH-C_2H_5$ | Cl | H | |
| 485 | $C_6H_5$ | $NH-nC_3H_7$ | Cl | H | |
| 486 | $C_6H_5$ | $NH-isoC_3H_7$ | Cl | H | |
| 487 | $C_6H_5$ | $NH-nC_4H_9$ | Cl | H | 97-98 |
| 488 | $C_6H_5$ | $NH-isoC_4H_9$ | Cl | H | |
| 489 | $C_6H_5$ | $NH-secC_4H_9$ | Cl | H | |
| 490 | $C_6H_5$ | $NH-tertC_4H_9$ | Cl | H | |
| 491 | $C_6H_5$ | $NH-nC_5H_{11}$ | Cl | H | 68-70 |
| 492 | $C_6H_5$ | $NH-NeoC_5H_{11}$ | Cl | H | |
| 493 | $C_6H_5$ | $NH-nC_6H_{13}$ | Cl | H | |
| 494 | $C_6H_5$ | $NH-nC_7H_{15}$ | Cl | H | |
| 495 | $C_6H_5$ | $NH-nC_8H_{17}$ | Cl | H | |
| 496 | $C_6H_5$ | $NH-nC_9H_{19}$ | Cl | H | |
| 497 | $C_6H_5$ | $NH-nC_{10}H_{21}$ | Cl | H | Öl |
| 498 | $C_6H_5$ | $NH-nC_{11}H_{23}$ | Cl | H | Öl |
| 499 | $C_6H_5$ | $NH-nC_{12}H_{25}$ | Cl | H | Öl |
| 500 | $C_6H_5$ | $NH-nC_{13}H_{27}$ | Cl | H | Öl |
| 501 | $C_6H_5$ | $NH-nC_{14}H_{29}$ | Cl | H | Öl |
| 502 | $C_6H_5$ | $NH-nC_{15}H_{31}$ | Cl | H | Öl |
| 503 | $C_6H_5$ | $NH-nC_{16}H_{33}$ | Cl | H | Öl |
| 504 | $C_6H_5$ | $NH-nC_{17}H_{35}$ | Cl | H | Öl |
| 505 | $C_6H_5$ | $NH-nC_{18}H_{37}$ | Cl | H | Öl |
| 506 | $C_6H_5$ | $NH-nC_{19}H_{39}$ | Cl | H | Öl |
| 507 | $C_6H_5$ | $NH-nC_{20}H_{41}$ | Cl | H | Öl |
| 508 | $C_6H_5$ | $NH-CH(CH_3)CH_2CH_2CH_3$ | Cl | H | Öl |
| 509 | $C_6H_5$ | $NH-CH(CH_3)-(CH_2)_3CH_3$ | Cl | H | Öl |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 510 | $C_6H_5$ | $NH-C_6H_5$ | Cl | H | |
| 511 | $C_6H_5$ | $NH-CH_2C_6H_5$ | Cl | H | |
| 512 | $C_6H_5$ | $NH-CH_2CH_2C_6H_5$ | Cl | H | |
| 513 | $C_6H_5$ | $NH-CH(CH_3)-C_6H_5$ | Cl | H | |
| 514 | $C_6H_5$ | $NH-(CH_2)_3-C_6H_5$ | Cl | H | |
| 515 | $C_6H_5$ | $NH-CH_2-COOH$ | Cl | H | |
| 516 | $C_6H_5$ | $NH-(CH_2)_2-COOH$ | Cl | H | |
| 517 | $C_6H_5$ | $NH-(CH_2)_3-COOH$ | Cl | H | |
| 518 | $C_6H_5$ | $NH-CH(CH_3)-(CH_2)_3-CH(CH_3)_2$ | Cl | H | Öl |
| 519 | $C_6H_5$ | $NH-(CH_2)_2-OH$ | Cl | H | |
| 520 | $C_6H_5$ | $NH-(CH_2)_3-OH$ | Cl | H | |
| 521 | $C_6H_5$ | $NH-(CH_2)_2-OCH_3$ | Cl | H | |
| 522 | $C_6H_5$ | $NH-(CH_2)_2-OC_2H_5$ | Cl | H | |
| 523 | $C_6H_5$ | $NH-(CH_2)_3-OCH_3$ | Cl | H | |
| 524 | $C_6H_5$ | $NH-(CH_2)_3-OC_2H_5$ | Cl | H | |
| 525 | $C_6H_5$ | NH-Cyclopropyl | Cl | H | |
| 526 | $C_6H_5$ | NH-Cyclopentyl | Cl | H | |
| 527 | $C_6H_5$ | NH-Cyclohexyl | Cl | H | |
| 528 | $C_6H_5$ | NH-Cycloheptyl | Cl | H | |
| 529 | $C_6H_5$ | NH-Cyclooctyl | Cl | H | |
| 530 | $C_6H_5$ | NH-Cyclododecyl | Cl | H | |
| 531 | H | $NH-CH_2-CH=CH_2$ | Cl | CHO | |
| 532 | H | $NH-CH_2-C\equiv CH$ | Cl | CHO | |
| 533 | H | NH-Methallyl | Cl | CHO | |
| 534 | $C_6H_5$ | $NH-CH_2-CH=CH_2$ | Cl | H | |
| 535 | $C_6H_5$ | $NH-CH_2-C\equiv CH$ | Cl | H | |
| 536 | $C_6H_5$ | NH-Methallyl | Cl | H | |
| 537 | H | $NH-CH_3$ | Cl | $COOC_2H_5$ | |
| 538 | H | $NH-C_2H_5$ | Cl | $COOC_2H_5$ | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 539 | H | $NH-nC_3H_7$ | Cl | $COOC_2H_5$ | |
| 540 | H | $NH-isoC_3H_7$ | Cl | $COOC_2H_5$ | |
| 541 | H | $NH-nC_4H_9$ | Cl | $COOC_2H_5$ | |
| 542 | H | $NH-isoC_4H_9$ | Cl | $COOC_2H_5$ | |
| 543 | H | $NH-secC_4H_9$ | Cl | $COOC_2H_5$ | |
| 544 | H | $NH-tertC_4H_9$ | Cl | $COOC_2H_5$ | |
| 545 | H | $NH-nC_5H_{11}$ | Cl | $COOC_2H_5$ | |
| 546 | H | $NH-NeoC_5H_{11}$ | Cl | $COOC_2H_5$ | |
| 547 | H | $NH-nC_6H_{13}$ | Cl | $COOC_2H_5$ | |
| 548 | H | $NH-nC_7H_{15}$ | Cl | $COOC_2H_5$ | |
| 549 | H | $NH-nC_8H_{17}$ | Cl | $COOC_2H_5$ | |
| 550 | H | $NH-nC_9H_{19}$ | Cl | $COOC_2H_5$ | |
| 551 | H | $NH-nC_{10}H_{21}$ | Cl | $COOC_2H_5$ | Öl |
| 552 | H | $NH-nC_{11}H_{23}$ | Cl | $COOC_2H_5$ | Öl |
| 553 | H | $NH-nC_{12}H_{25}$ | Cl | $COOC_2H_5$ | Öl |
| 554 | H | $NH-nC_{13}H_{27}$ | Cl | $COOC_2H_5$ | Öl |
| 555 | H | $NH-nC_{14}H_{29}$ | Cl | $COOC_2H_5$ | Öl |
| 556 | H | $NH-nC_{15}H_{31}$ | Cl | $COOC_2H_5$ | Öl |
| 557 | H | $NH-nC_{16}H_{33}$ | Cl | $COOC_2H_5$ | Öl |
| 558 | H | $NH-nC_{17}H_{35}$ | Cl | $COOC_2H_5$ | Öl |
| 559 | H | $NH-nC_{18}H_{37}$ | Cl | $COOC_2H_5$ | Öl |
| 560 | H | $NH-nC_{19}H_{39}$ | Cl | $COOC_2H_5$ | Öl |
| 561 | H | $NH-nC_{20}H_{41}$ | Cl | $COOC_2H_5$ | Öl |
| 562 | H | $NH-CH(CH_3)CH_2CH_2CH_3$ | Cl | $COOC_2H_5$ | Öl |
| 563 | H | $NH-CH(CH_3)-(CH_2)_3CH_3$ | Cl | $COOC_2H_5$ | Öl |
| 564 | H | $NH-C_6H_5$ | Cl | $COOC_2H_5$ | |
| 565 | H | $NH-CH_2C_6H_5$ | Cl | $COOC_2H_5$ | |
| 566 | H | $NH-CH_2CH_2C_6H_5$ | Cl | $COOC_2H_5$ | |
| 567 | H | $NH-CH(CH_3)-C_6H_5$ | Cl | $COOC_2H_5$ | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 568 | H | $NH-(CH_2)_3-C_6H_5$ | Cl | $COOC_2H_5$ | |
| 569 | H | $NH-CH_2-COOH$ | Cl | $COOC_2H_5$ | |
| 570 | H | $NH-(CH_2)_2-COOH$ | Cl | $COOC_2H_5$ | |
| 571 | H | $NH-(CH_2)_3-COOH$ | Cl | $COOC_2H_5$ | |
| 572 | H | $NH-CH(CH_3)-(CH_2)_3$ $CH(CH_3)_2$ | Cl | $COOC_2H_5$ | Öl |
| 573 | H | $NH-(CH_2)_2-OH$ | Cl | $COOC_2H_5$ | |
| 574 | H | $NH-(CH_2)_3-OH$ | Cl | $COOC_2H_5$ | |
| 575 | H | $NH-(CH_2)_2-OCH_3$ | Cl | $COOC_2H_5$ | |
| 576 | H | $NH-(CH_2)_2-OC_2H_5$ | Cl | $COOC_2H_5$ | |
| 577 | H | $NH-(CH_2)_3-OCH_3$ | Cl | $COOC_2H_5$ | |
| 578 | H | $NH-(CH_2)_3-OC_2H_5$ | Cl | $COOC_2H_5$ | |
| 579 | H | NH-Cyclopropyl | Cl | $COOC_2H_5$ | |
| 580 | H | NH-Cyclopentyl | Cl | $COOC_2H_5$ | |
| 581 | H | NH-Cyclohexyl | Cl | $COOC_2H_5$ | |
| 582 | H | NH-Cycloheptyl | Cl | $COOC_2H_5$ | |
| 583 | H | NH-Cyclooctyl | Cl | $COOC_2H_5$ | |
| 584 | H | NH-Cyclododecyl | Cl | $COOC_2H_5$ | |
| 585 | H | $NH-CH_2-CH=CH_2$ | Cl | $COOC_2H_5$ | |
| 586 | H | $NH-CH_2-C\equiv CH$ | Cl | $COOC_2H_5$ | |
| 587 | H | NH-Methallyl | Cl | $COOC_2H_5$ | |
| 588 | H | $NH-CH_3$ | Cl | $CONH_2$ | |
| 589 | H | $NH-C_2H_5$ | Cl | $CONH_2$ | |
| 590 | H | $NH-nC_3H_7$ | Cl | $CONH_2$ | |
| 591 | H | $NH-isoC_3H_7$ | Cl | $CONH_2$ | |
| 592 | H | $NH-nC_4H_9$ | Cl | $CONH_2$ | |
| 593 | H | $NH-isoC_4H_9$ | Cl | $CONH_2$ | |
| 594 | H | $NH-secC_4H_9$ | Cl | $CONH_2$ | |
| 595 | H | $NH-tertC_4H_9$ | Cl | $CONH_2$ | |
| 596 | H | $NH-nC_5H_{11}$ | Cl | $CONH_2$ | |

Tabelle 1 (Fortsetzung)

| Bsp. Nr. | Rest $R^1$ | Rest $R^2$ | Rest $R^3$ | Rest $R^4$ | Schmelz-punkt (°C) |
|---|---|---|---|---|---|
| 597 | H | NH-Neo$C_5H_{11}$ | Cl | $CONH_2$ | |
| 598 | H | NH-n$C_6H_{13}$ | Cl | $CONH_2$ | |
| 599 | H | NH-n$C_7H_{15}$ | Cl | $CONH_2$ | |
| 600 | H | NH-n$C_8H_{17}$ | Cl | $CONH_2$ | |
| 601 | H | NH-n$C_9H_{19}$ | Cl | $CONH_2$ | |
| 602 | H | NH-n$C_{10}H_{21}$ | Cl | $CONH_2$ | |
| 603 | H | NH-n$C_{11}H_{23}$ | Cl | $CONH_2$ | Öl |
| 604 | H | NH-n$C_{12}H_{25}$ | Cl | $CONH_2$ | Öl |
| 605 | H | NH-n$C_{13}H_{27}$ | Cl | $CONH_2$ | Öl |
| 606 | H | NH-n$C_{14}H_{29}$ | Cl | $CONH_2$ | Öl |
| 607 | H | NH-n$C_{15}H_{31}$ | Cl | $CONH_2$ | Öl |
| 608 | H | NH-n$C_{16}H_{33}$ | Cl | $CONH_2$ | Öl |
| 609 | H | NH-n$C_{17}H_{35}$ | Cl | $CONH_2$ | Öl |
| 610 | H | NH-n$C_{18}H_{37}$ | Cl | $CONH_2$ | Öl |
| 611 | H | NH-n$C_{19}H_{39}$ | Cl | $CONH_2$ | Öl |
| 612 | H | NH-n$C_{20}H_{41}$ | Cl | $CONH_2$ | Öl |
| 613 | H | NH-$CH(CH_3)CH_2CH_2CH_3$ | Cl | $CONH_2$ | Öl |
| 614 | H | NH-$CH(CH_3)-(CH_2)_3CH_3$ | Cl | $CONH_2$ | Öl |
| 615 | H | NH-$C_6H_5$ | Cl | $CONH_2$ | |
| 616 | H | NH-$CH_2C_6H_5$ | Cl | $CONH_2$ | |
| 617 | H | NH-$CH_2CH_2C_6H_5$ | Cl | $CONH_2$ | |
| 618 | H | NH-$CH(CH_3)-C_6H_5$ | Cl | $CONH_2$ | |
| 619 | H | NH-$(CH_2)_3-C_6H_5$ | Cl | $CONH_2$ | |
| 620 | H | NH-$CH_2$-COOH | Cl | $CONH_2$ | |
| 621 | H | NH-$(CH_2)_2$-COOH | Cl | $CONH_2$ | |
| 622 | H | NH-$(CH_2)_3$-COOH | Cl | $CONH_2$ | |
| 623 | H | NH-$CH(CH_3)-(CH_2)_3-CH(CH_3)_2$ | Cl | $CONH_2$ | Öl |
| 624 | H | NH-$(CH_2)_2$-OH | Cl | $CONH_2$ | |
| 625 | H | NH-$(CH_2)_3$-OH | Cl | $CONH_2$ | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 626 | H | $NH-(CH_2)_2-OCH_3$ | Cl | $CONH_2$ | |
| 627 | H | $NH-(CH_2)_2-OC_2H_5$ | Cl | $CONH_2$ | |
| 628 | H | $NH-(CH_2)_3-OCH_3$ | Cl | $CONH_2$ | |
| 629 | H | $NH-(CH_2)_3-OC_2H_5$ | Cl | $CONH_2$ | |
| 630 | H | NH-Cyclopropyl | Cl | $CONH_2$ | |
| 631 | H | NH-Cyclopentyl | Cl | $CONH_2$ | |
| 632 | H | NH-Cyclohexyl | Cl | $CONH_2$ | |
| 633 | H | NH-Cycloheptyl | Cl | $CONH_2$ | |
| 634 | H | NH-Cyclooctyl | Cl | $CONH_2$ | |
| 635 | H | NH-Cyclododecyl | Cl | $CONH_2$ | |
| 636 | H | $NH-CH_2-CH=CH_2$ | Cl | $CONH_2$ | |
| 637 | H | $NH-CH_2-C \equiv CH$ | Cl | $CONH_2$ | |
| 638 | H | NH-Methallyl | Cl | $CONH_2$ | |
| 639 | H | $NH-CH_3$ | Cl | $COOC_2H_5$ | |
| 640 | H | $NH-C_2H_5$ | Cl | $COOC_2H_5$ | |
| 641 | H | $NH-nC_3H_7$ | Cl | $COOC_2H_5$ | |
| 642 | H | $NH-isoC_3H_7$ | Cl | $COOC_2H_5$ | |
| 643 | H | $NH-nC_4H_9$ | Cl | $COOC_2H_5$ | |
| 644 | H | $NH-isoC_4H_9$ | Cl | $COOC_2H_5$ | |
| 645 | H | $NH-secC_4H_9$ | Cl | $COOC_2H_5$ | |
| 646 | H | $NH-tertC_4H_9$ | Cl | $COOC_2H_5$ | |
| 647 | H | $NH-nC_5H_{11}$ | Cl | $COOC_2H_5$ | |
| 648 | H | $NH-NeoC_5H_{11}$ | Cl | $COOC_2H_5$ | |
| 649 | H | $NH-nC_6H_{13}$ | Cl | $COOC_2H_5$ | |
| 650 | H | $NH-nC_7H_{15}$ | Cl | $COOC_2H_5$ | |
| 651 | H | $NH-nC_8H_{17}$ | Cl | $COOC_2H_5$ | |
| 652 | H | $NH-nC_9H_{19}$ | Cl | $COOC_2H_5$ | |
| 653 | H | $NH-nC_{10}H_{21}$ | Cl | $COOC_2H_5$ | Öl |
| 654 | H | $NH-nC_{11}H_{23}$ | Cl | $COOC_2H_5$ | Öl |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 655 | H | $NH-nC_{12}H_{25}$ | Cl | $COOC_2H_5$ | Öl |
| 656 | H | $NH-nC_{13}H_{27}$ | Cl | $COOC_2H_5$ | Öl |
| 657 | H | $NH-nC_{14}H_{29}$ | Cl | $COOC_2H_5$ | Öl |
| 658 | H | $NH-nC_{15}H_{31}$ | Cl | $COOC_2H_5$ | Öl |
| 659 | H | $NH-nC_{16}H_{33}$ | Cl | $COOC_2H_5$ | Öl |
| 660 | H | $NH-nC_{17}H_{35}$ | Cl | $COOC_2H_5$ | Öl |
| 661 | H | $NH-nC_{18}H_{37}$ | Cl | $COOC_2H_5$ | Öl |
| 662 | H | $NH-nC_{19}H_{39}$ | Cl | $COOC_2H_5$ | Öl |
| 663 | H | $NH-nC_{20}H_{41}$ | Cl | $COOC_2H_5$ | Öl |
| 664 | H | $NH-CH(CH_3)CH_2CH_2CH_3$ | Cl | $COOC_2H_5$ | Öl |
| 665 | H | $NH-CH(CH_3)-(CH_2)_3CH_3$ | Cl | $COOC_2H_5$ | Öl |
| 666 | H | $NH-C_6H_5$ | Cl | $COOC_2H_5$ | |
| 667 | H | $NH-CH_2C_6H_5$ | Cl | $COOC_2H_5$ | |
| 668 | H | $NH-CH_2CH_2C_6H_5$ | Cl | $COOC_2H_5$ | |
| 669 | H | $NH-CH(CH_3)-C_6H_5$ | Cl | $COOC_2H_5$ | |
| 670 | H | $NH-(CH_2)_3-C_6H_5$ | Cl | $COOC_2H_5$ | |
| 671 | H | $NH-CH_2-COOH$ | Cl | $COOC_2H_5$ | |
| 672 | H | $NH-(CH_2)_2-COOH$ | Cl | $COOC_2H_5$ | |
| 673 | H | $NH-(CH_2)_3-COOH$ | Cl | $COOC_2H_5$ | |
| 674 | H | $NH-CH(CH_3)-(CH_2)_3-CH(CH_3)_2$ | Cl | $COOC_2H_5$ | Öl |
| 675 | H | $NH-(CH_2)_2-OH$ | Cl | $COOC_2H_5$ | |
| 676 | H | $NH-(CH_2)_3-OH$ | Cl | $COOC_2H_5$ | |
| 677 | H | $NH-(CH_2)_2-OCH_3$ | Cl | $COOC_2H_5$ | |
| 678 | H | $NH-(CH_2)_2-OC_2H_5$ | Cl | $COOC_2H_5$ | |
| 679 | H | $NH-(CH_2)_3-OCH_3$ | Cl | $COOC_2H_5$ | |
| 680 | H | $NH-(CH_2)_3-OC_2H_5$ | Cl | $COOC_2H_5$ | |
| 681 | H | NH-Cyclopropyl | Cl | $COOC_2H_5$ | |
| 682 | H | NH-Cyclopentyl | Cl | $COOC_2H_5$ | |
| 683 | H | NH-Cyclohexyl | Cl | $COOC_2H_5$ | |

Tabelle 1 (Fortsetzung)

| Bsp. Nr. | Rest $R^1$ | Rest $R^2$ | Rest $R^3$ | Rest $R^4$ | Schmelz-punkt (°C) |
|---|---|---|---|---|---|
| 684 | H | NH–Cycloheptyl | Cl | $COOC_2H_5$ | |
| 685 | H | NH–Cyclooctyl | Cl | $COOC_2H_5$ | |
| 686 | H | NH–Cyclododecyl | Cl | $COOC_2H_5$ | |
| 687 | H | $NH-CH_2-CH=CH_2$ | Cl | $COOC_2H_5$ | |
| 688 | H | $NH-CH_2-C\equiv CH$ | Cl | $COOC_2H_5$ | |
| 689 | H | NH–Methallyl | Cl | $COOC_2H_5$ | |
| 690 | H | $NH-CH_3$ | Cl | $COOCH_3$ | |
| 691 | H | $NH-C_2H_5$ | Cl | $COOCH_3$ | |
| 692 | H | $NH-nC_3H_7$ | Cl | $COOCH_3$ | |
| 693 | H | $NH-isoC_3H_7$ | Cl | $COOCH_3$ | |
| 694 | H | $NH-nC_4H_9$ | Cl | $COOCH_3$ | |
| 695 | H | $NH-isoC_4H_9$ | Cl | $COOCH_3$ | |
| 696 | H | $NH-secC_4H_9$ | Cl | $COOCH_3$ | |
| 697 | H | $NH-tertC_4G_9$ | Cl | $COOCH_3$ | |
| 698 | H | $NH-nC_5H_{11}$ | Cl | $COOCH_3$ | |
| 699 | H | $NH-NeoC_5H_{11}$ | Cl | $COOCH_3$ | |
| 700 | H | $NH-nC_6H_{13}$ | Cl | $COOCH_3$ | |
| 701 | H | $NH-nC_7H_{15}$ | Cl | $COOCH_3$ | |
| 702 | H | $NH-nC_8H_{17}$ | Cl | $COOCH_3$ | |
| 703 | H | $NH-nC_9H_{19}$ | Cl | $COOCH_3$ | |
| 704 | H | $NH-nC_{10}H_{21}$ | Cl | $COOCH_3$ | Öl |
| 705 | H | $NH-nC_{11}H_{23}$ | Cl | $COOCH_3$ | Öl |
| 706 | H | $NH-nC_{12}H_{25}$ | Cl | $COOCH_3$ | Öl |
| 707 | H | $NH-nC_{13}H_{27}$ | Cl | $COOCH_3$ | Öl |
| 708 | H | $NH-nC_{14}H_{29}$ | Cl | $COOCH_3$ | Öl |
| 709 | H | $NH-nC_{15}H_{31}$ | Cl | $COOCH_3$ | Öl |
| 710 | H | $NH-nC_{16}H_{33}$ | Cl | $COOCH_3$ | Öl |
| 711 | H | $NH-nC_{17}H_{35}$ | Cl | $COOCH_3$ | Öl |
| 712 | H | $NH-nC_{18}H_{37}$ | Cl | $COOCH_3$ | Öl |
| 713 | H | $NH-nC_{19}H_{39}$ | Cl | $COOCH_3$ | Öl |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 714 | H | $NH-nC_{20}H_{41}$ | Cl | $COOCH_3$ | Öl |
| 715 | H | $NH-CH(CH_3)CH_2CH_2CH_3$ | Cl | $COOCH_3$ | Öl |
| 716 | H | $NH-CH(CH_3)-(CH_2)_3CH_3$ | Cl | $COOCH_3$ | Öl |
| 717 | H | $NH-C_6H_5$ | Cl | $COOCH_3$ | |
| 718 | H | $NH-CH_2C_6H_5$ | Cl | $COOCH_3$ | |
| 719 | H | $NH-CH_2CH_2C_6H_5$ | Cl | $COOCH_3$ | |
| 720 | H | $NH-CH(CH_3)-C_6H_5$ | Cl | $COOCH_3$ | |
| 721 | H | $NH-(CH_2)_3-C_6H_5$ | Cl | $COOCH_3$ | |
| 722 | H | $NH-CH_2-COOH$ | Cl | $COOCH_3$ | |
| 723 | H | $NH-(CH_2)_2-COOH$ | Cl | $COOCH_3$ | |
| 724 | H | $NH-(CH_2)_3-COOH$ | Cl | $COOCH_3$ | |
| 725 | H | $NH-(CH(CH_3)-(CH_2)_3-CH(CH_3)_2$ | Cl | $COOCH_3$ | Öl |
| 726 | H | $NH-(CH_2)_2-OH$ | Cl | $COOCH_3$ | |
| 727 | H | $NH-(CH_2)_3-OH$ | Cl | $COOCH_3$ | |
| 728 | H | $NH-(CH_2)_2-OCH_3$ | Cl | $COOCH_3$ | |
| 729 | H | $NH-(CH_2)_2-OC_2H_5$ | Cl | $COOCH_3$ | |
| 730 | H | $NH-(CH_2)_3-OCH_3$ | Cl | $COOCH_3$ | |
| 731 | H | $NH-(CH_2)_3-OCH_5$ | Cl | $COOCH_3$ | |
| 732 | H | NH–Cyclopropyl | Cl | $COOCH_3$ | |
| 733 | H | NH–Cyclopentyl | Cl | $COOCH_3$ | |
| 734 | H | NH–Cyclohexyl | Cl | $COOCH_3$ | |
| 735 | H | NH–Cycloheptyl | Cl | $COOCH_3$ | |
| 736 | H | NH–Cyclooctyl | Cl | $COOCH_3$ | |
| 737 | H | NH–Cyclododecyl | Cl | $COOCH_3$ | |
| 738 | H | $NH-CH_2-CH=CH_2$ | Cl | $COOCH_3$ | |
| 739 | H | $NH-CH_2-C\equiv CH_2$ | Cl | $COOCH_3$ | |
| 740 | H | NH–Methallyl | Cl | $COOCH_3$ | |
| 741 | H | $NH-nC_6H_{13}$ | Cl | COOH | |
| 742 | H | $NH-nC_8H_{17}$ | Cl | COOH | |
| 743 | H | $NH-nC_{10}H_{21}$ | Cl | COOH | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 744 | $CH_3$ | $NH-nC_6H_{13}$ | Cl | CHO | |
| 745 | $CH_3$ | $NH-nC_8H_{17}$ | Cl | CHO | |
| 746 | $CH_3$ | $NH-nC_{10}H_{21}$ | Cl | CHO | |
| 747 | $C_6H_5$ | $NH-nC_6H_{13}$ | Cl | CHO | |
| 748 | $C_6H_5$ | $NH-nC_8H_{17}$ | Cl | CHO | |
| 749 | $C_6H_5$ | $NH-nC_{10}H_{21}$ | Cl | CHO | |
| 750 | $CH_2Cl_2$ | Cl | Cl | H | 97-99 |
| 751 | H | $CH(CH_3)-(CH_2)_4CH_3$ | Cl | CHO | öl |
| 752 | H | $NH-C_6H_4-(2)-CH_3$ | Cl | CHO | |
| 753 | H | $NH-C_6H_4-(2)-C_2H_5$ | Cl | CHO | |
| 754 | H | $NH-C_6H_4-(2)-CF_3$ | Cl | CHO | |
| 755 | H | $NH-(CH_2)_3-isoC_3H_7$ | Cl | CHO | |
| 756 | H | $NH-(CH_2)_3-CH_2C_6H_5$ | Cl | CHO | |
| 757 | H | $N(CH_3)_2$ | Cl | CHO | |
| 758 | H | $N(C_2H_5)_2$ | Cl | CHO | |
| 759 | H | $N(nC_3H_7)_2$ | Cl | CHO | |
| 760 | H | $N(nC_4H_9)_2$ | Cl | CHO | |
| 761 | H | $N(CH_2CH=CH_2)_2$ | Cl | CHO | |
| 762 | H | Pyrrolidino | Cl | CHO | |
| 763 | H | Piperidino | Cl | CHO | |
| 764 | H | Hexamethylenimino | Cl | CHO | |
| 765 | H | Morpholino | Cl | CHO | |
| 766 | H | $N(CH_2CH_2OCH_3)_2$ | Cl | CHO | öl |
| 767 | H | $S-CH_3$ | Cl | CHO | |
| 768 | H | $S-C_2H_5$ | Cl | CHO | |
| 769 | H | $S-nC_3H_7$ | Cl | CHO | |
| 770 | H | $S-CH_2-CH=CH_3$ | Cl | CHO | |
| 771 | H | SCN | Cl | CHO | |
| 772 | H | $O-CH_3$ | Cl | CHO | |
| 773 | H | $O-C_2H_5$ | Cl | CHO | |
| 774 | H | $O-nC_3H_7$ | Cl | CHO | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 775 | H | $O-CH_2CH_2OCH_3$ | Cl | CHO | |
| 776 | H | $O-CH_2CH(CH_3)OCH_3$ | Cl | CHO | |
| 777 | $C_6H_5$ | $CH(CH_3)-(CH_2)_4CH_3$ | Cl | H | Öl |
| 778 | $C_6H_5$ | $NH-C_6H_4-(2)-CH_3$ | Cl | H | |
| 779 | $C_6H_5$ | $NH-C_6H_4-(2)-C_2H_5$ | Cl | H | |
| 780 | $C_6H_5$ | $NH-C_6H_4-(2)-CF_3$ | Cl | H | |
| 781 | $C_6H_5$ | $NH-(CH_2)_3-isoC_3H_7$ | Cl | H | |
| 782 | $C_6H_5$ | $NH-(CH_2)_3-CH_2C_6H_5$ | Cl | H | |
| 783 | $C_6H_5$ | $N(CH_3)_2$ | Cl | H | |
| 784 | $C_6H_5$ | $N(C_2H_5)_2$ | Cl | H | |
| 785 | $C_6H_5$ | $N(C_2H_5)_2$ | Cl | H | |
| 786 | $C_6H_5$ | $N(nC_4H_9)_2$ | Cl | H | |
| 787 | $C_6H_5$ | $N(CH_2CH=CH_2)_2$ | Cl | H | |
| 788 | $C_6H_5$ | Pyrrolidino | Cl | H | |
| 789 | $C_6H_5$ | Piperidino | Cl | H | |
| 790 | $C_6H_5$ | Hexamethylenimino | Cl | H | |
| 791 | $C_6H_5$ | Morpholino | Cl | H | |
| 792 | $C_6H_5$ | $N(CH_2CH_2OCH_3)_2$ | Cl | H | |
| 793 | $C_6H_5$ | $S-CH_3$ | Cl | H | |
| 794 | $C_6H_5$ | $S-C_2H_5$ | Cl | H | |
| 795 | $C_6H_5$ | $S-nC_3H_7$ | Cl | H | |
| 796 | $C_6H_5$ | $S-CH_2-CH=CH_3$ | Cl | H | |
| 797 | $C_6H_5$ | SCN | Cl | H | |
| 798 | $C_6H_5$ | $O-CH_3$ | Cl | H | |
| 799 | $C_6H_5$ | $O-C_2H_5$ | Cl | H | |
| 800 | $C_6H_5$ | $O-nC_3H_7$ | Cl | H | |
| 801 | $C_6H_5$ | $O-CH_2CH_2OCH_3$ | Cl | H | |
| 802 | $C_6H_5$ | $O-CH_2CH(CH_3)OCH_3$ | Cl | H | |
| 803 | H | $NH-CH(CH_3)-(CH_2)_4CH_3$ | Cl | $CONH_2$ | Öl |
| 804 | H | $NH-C_6H_4-(2)-CH_3$ | Cl | $CONH_2$ | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 805 | H | $NH-C_6H_4-(2)-C_2H_5$ | Cl | $CONH_2$ | |
| 806 | H | $NH-C_6H_4-(2)-CF_3$ | Cl | $CONH_2$ | |
| 807 | H | $NH-(CH_2)_3-isoC_3H_7$ | Cl | $CONH_2$ | |
| 808 | H | $NH-(CH_2)_3-CH_2C_6H_5$ | Cl | $CONH_2$ | |
| 809 | H | $N(CH_3)_2$ | Cl | $CONH_2$ | |
| 810 | H | $N(C_2H_5)_2$ | Cl | $CONH_2$ | |
| 811 | H | $N(nC_3H_7)_2$ | Cl | $CONH_2$ | |
| 812 | H | $N(nC_4H_9)_2$ | Cl | $CONH_2$ | |
| 813 | H | $N(CH_2CH=CH_2)_2$ | Cl | $CONH_2$ | |
| 814 | H | Pyrrolidino | Cl | $CONH_2$ | |
| 815 | H | Piperidino | Cl | $CONH_2$ | |
| 816 | H | Hexamethylenimino | Cl | $CONH_2$ | |
| 817 | H | Morpholino | Cl | $CONH_2$ | |
| 818 | H | $N(CH_2CH_2OCH_3)_2$ | Cl | $CONH_2$ | |
| 819 | H | $S-CH_3$ | Cl | $CONH_2$ | |
| 820 | H | $S-C_2H_5$ | Cl | $CONH_2$ | |
| 821 | H | $S-nC_3H_7$ | Cl | $CONH_2$ | |
| 822 | H | $S-CH_2-CH=CH_3$ | Cl | $CONH_2$ | |
| 823 | H | SCN | Cl | $CONH_2$ | |
| 824 | H | $O-CH_3$ | Cl | $CONH_2$ | |
| 825 | H | $O-C_2H_5$ | Cl | $CONH_2$ | |
| 826 | H | $O-nC_3H_7$ | Cl | $CONH_2$ | |
| 827 | H | $O-CH_2CH_2OCH_3$ | Cl | $CONH_2$ | |
| 828 | H | $O-CH_2CH(CH_3)OCH_3$ | Cl | $CONH_2$ | |
| 829 | H | $CH(CH_3)-(CH_2)_4CH_3$ | Cl | COOH | Öl |
| 830 | H | $NH-C_6H_4-(2)-CH_3$ | Cl | COOH | |
| 831 | H | $NH-C_6H_4-(2)-C_2H_5$ | Cl | COOH | |
| 832 | H | $NH-C_6H_4-(2)-CF_3$ | Cl | COOH | |
| 833 | H | $NH-(CH_2)_3-isoC_3H_7$ | Cl | COOH | |
| 834 | H | $NH-(CH_2)_3-CH_2C_6H_5$ | Cl | COOH | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 835 | H | $N(CH_3)_2$ | Cl | COOH | |
| 836 | H | $N(C_2H_5)_2$ | Cl | COOH | |
| 837 | H | $N(nC_3H_7)_2$ | Cl | COOH | |
| 838 | H | $N(nC_4H_9)_2$ | Cl | COOH | |
| 839 | H | $N(CH_2CH{=}CH_2)_2$ | Cl | COOH | |
| 840 | H | Pyrrolidino | Cl | COOH | |
| 841 | H | Piperidino | Cl | COOH | |
| 842 | H | Hexamethylenimino | Cl | COOH | |
| 843 | H | Morpholino | Cl | COOH | |
| 844 | H | $N(CH_2CH_2OCH_3)_2$ | Cl | COOH | |
| 845 | H | $S{-}CH_3$ | Cl | COOH | |
| 846 | H | $S{-}C_2H_5$ | Cl | COOH | |
| 847 | H | $S{-}nC_3H_7$ | Cl | COOH | |
| 848 | H | $S{-}CH_2{-}CH{=}CH_3$ | Cl | COOH | |
| 849 | H | SCN | Cl | COOH | |
| 850 | H | $O{-}CH_3$ | Cl | COOH | |
| 851 | H | $O{-}C_2H_5$ | Cl | COOH | |
| 852 | H | $O{-}nC_3H_7$ | Cl | COOH | |
| 853 | H | $O{-}CH_2CH_2OCH_3$ | Cl | COOH | |
| 854 | H | $O{-}CH_2CH(CH_3)OCH_3$ | Cl | COOH | |
| 855 | H | $CH(CH_3){-}(CH_2)_4CH_3$ | Cl | $COOCH_3$ | |
| 856 | H | $NH{-}C_6H_4{-}(2){-}CH_3$ | Cl | $COOCH_3$ | |
| 857 | H | $NH{-}C_6H_4{-}(2){-}C_2H_5$ | Cl | $COOCH_3$ | |
| 858 | H | $NH{-}C_6H_4{-}(2){-}CF_3$ | Cl | $COOCH_3$ | |
| 859 | H | $NH{-}(CH_2)_3{-}isoC_3H_7$ | Cl | $COOCH_3$ | |
| 860 | H | $NH{-}(CH_2)_3{-}CH_2C_6H_5$ | Cl | $COOCH_3$ | |
| 861 | H | $N(CH_3)_2$ | Cl | $COOCH_3$ | |
| 862 | H | $N(C_2H_5)_2$ | Cl | $COOCH_3$ | |
| 863 | H | $N(nC_3H_7)_2$ | Cl | $COOCH_3$ | |
| 864 | H | $N(nC_4H_9)_2$ | Cl | $COOCH_3$ | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 865 | H | $N(CH_2CH{=}CH_2)_2$ | Cl | $COOCH_3$ | |
| 866 | H | Pyrrolidino | Cl | $COOCH_3$ | |
| 867 | H | Piperidino | Cl | $COOCH_3$ | |
| 868 | H | Hexamethylenimino | Cl | $COOCH_3$ | |
| 869 | H | Morpholino | Cl | $COOCH_3$ | |
| 870 | H | $N(CH_2CH_2OCH_3)_2$ | Cl | $COOCH_3$ | |
| 871 | H | $S-CH_3$ | Cl | $COOCH_3$ | |
| 872 | H | $S-C_2H_5$ | Cl | $COOCH_3$ | |
| 873 | H | $S-nC_3H_7$ | Cl | $COOCH_3$ | |
| 874 | H | $S-CH_2-CH{=}CH_3$ | Cl | $COOCH_3$ | |
| 875 | H | SCN | Cl | $COOCH_3$ | |
| 876 | H | $O-CH_3$ | Cl | $COOCH_3$ | |
| 877 | H | $CH(CH_3)-(CH_2)_4CH_3$ | Cl | $COOC_2H_5$ | Öl |
| 878 | H | $NH-C_6H_4-(2)-CH_3$ | Cl | $COOC_2H_5$ | |
| 879 | H | $NH-C_6H_4-(2)-C_2H_5$ | Cl | $COOC_2H_5$ | |
| 880 | H | $NH-C_6H_4-(2)-CF_3$ | Cl | $COOC_2H_5$ | |
| 881 | H | $NH-(CH_2)_3-isoC_3H_7$ | Cl | $COOC_2H_5$ | |
| 882 | H | $NH-(CH_2)_3-CH_2C_6H_5$ | Cl | $COOC_2H_5$ | |
| 883 | H | $N(CH_3)_2$ | Cl | $COOC_2H_5$ | |
| 884 | H | $N(C_2H_5)_2$ | Cl | $COOC_2H_5$ | |
| 885 | H | $N(nC_3H_7)_2$ | Cl | $COOC_2H_5$ | |
| 886 | H | $N(nC_4H_9)2$ | Cl | $COOC_2H_5$ | |
| 887 | H | $N(CH_2CH{=}CH_2)_2$ | Cl | $COOC_2H_5$ | |
| 888 | H | Pyrrolidino | Cl | $COOC_2H_5$ | |
| 889 | H | Piperidino | Cl | $COOC_2H_5$ | |
| 890 | H | Hexamethylenimino | Cl | $COOC_2H_5$ | |
| 891 | H | Morpholino | Cl | $COOC_2H_5$ | |
| 892 | H | $N(CH_2CH_2OCH_3)_2$ | Cl | $COOC_2H_5$ | |
| 893 | H | $S-CH_3$ | Cl | $COOC_2H_5$ | |
| 894 | H | $S-C_2H_5$ | Cl | $COOC_2H_5$ | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 895 | H | $S-nC_3H_7$ | Cl | $COOC_2H_5$ | |
| 896 | H | $S-CH_2-CH=CH_3$ | Cl | $COOC_2H_5$ | |
| 897 | H | SCN | Cl | $COOC_2H_5$ | |
| 898 | H | $O-C_2H_5$ | Cl | $COOC_2H_5$ | |
| 899 | H | $O-nC_3H_7$ | Cl | $COOnC_3H_7$ | |
| 900 | H | $O-nC_4H_9$ | Cl | $COOnC_4H_9$ | |
| 901 | H | $O-nC_5H_{11}$ | Cl | $COOnC_5H_{11}$ | |
| 902 | H | $O-nC_6H_{13}$ | Cl | $COOnC_6H_{13}$ | |
| 903 | H | Cl | Cl | $C_6H_5$ | 262-266 |
| 904 | $CH_3$ | $NH_2$ | Cl | H | |
| 905 | $CH_3$ | $NH-nC_4H_9$ | Cl | H | 37-40 |
| 906 | $CH_3$ | $NH-isoC_4H_9$ | Cl | H | |
| 907 | $CH_3$ | $NH-tertC_4H_9$ | | | |
| 908 | $CH_3$ | $NH-isoC_5H_{11}$ | Cl | H | |
| 909 | $CH_3$ | $NH-NeoC_5H_{11}$ | Cl | H | |
| 910 | $CH_3$ | $NH-nC_6H_{13}$ | Cl | H | 59-60 |
| 911 | $CH_3$ | $NH-nC_7H_{15}$ | Cl | H | |
| 912 | $CH_3$ | $NH-nC_8H_{17}$ | Cl | H | |
| 913 | $CH_3$ | $NH-nC_9H_{19}$ | Cl | H | Öl |
| 914 | $CH_3$ | $NH-nC_{10}H_{21}$ | Cl | H | Öl |
| 915 | $CH_3$ | $NH-nC_{11}H_{23}$ | Cl | H | Öl |
| 916 | $CH_3$ | $NH-nC_{12}H_{25}$ | Cl | H | Öl |
| 917 | $CH_3$ | $NH-nC_{13}H_{27}$ | Cl | H | Öl |
| 918 | $CH_3$ | $NH-nC_{14}H_{29}$ | Cl | H | Öl |
| 919 | $CH_3$ | $NH-nC_{15}H_{31}$ | Cl | H | Öl |
| 920 | $CH_3$ | $NH-nC_{16}H_{33}$ | Cl | H | Öl |
| 921 | $CH_3$ | $NH-nC_{17}H_{35}$ | Cl | H | Öl |
| 922 | $CH_3$ | $NH-nC_{18}H_{37}$ | Cl | H | Öl |
| 923 | $CH_3$ | $NH-nC_{19}H_{39}$ | Cl | H | Öl |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 924 | $CH_3$ | $NH-nC_{20}H_{41}$ | Cl | H | Öl |
| 925 | $CH_3$ | $NH-CH(CH_3)CH_2CH_2CH_3$ | Cl | H | Öl |
| 926 | $CH_3$ | $NH-CH(CH_3)-$ $-(CH_2)_3-CH_3$ | Cl | H | Öl |
| 927 | $CH_3$ | $NH-CH_2C_6H_5$ | Cl | H | |
| 928 | $CH_3$ | $NH-CH_2CH_2C_6H_5$ | Cl | H | |
| 929 | $CH_3$ | $NH-CH(CH_3)-C_6H_5$ | Cl | H | |
| 930 | $CH_3$ | $NH-(CH_2)_3-C_6H_5$ | Cl | H | |
| 931 | $CH_3$ | $NH-CH_2-COOH$ | Cl | H | |
| 932 | $CH_3$ | $NH-(CH_2)_2-COOH$ | Cl | H | |
| 933 | $CH_3$ | $NH-(CH_2)_3-COOH$ | Cl | H | |
| 934 | $CH_3$ | $NH-CH(CH_3)-$ $-(CH_2)_3-CH(CH_3)_2$ | Cl | H | |
| 935 | $CH_3$ | $NH-(CH_2)_2-OH$ | Cl | H | |
| 936 | $CH_3$ | $NH-(CH_2)_2-OCH_3$ | Cl | H | |
| 937 | $CH_3$ | $NH-(CH_2)_2-OC_2H_5$ | Cl | H | |
| 938 | $CH_3$ | $NH-(CH_2)_3-OCH_3$ | Cl | H | |
| 939 | $CH_3$ | $NH-(CH_2)_3-OC_2H_5$ | Cl | H | |
| 940 | $CH_3$ | NH—Cyclopropyl | Cl | H | |
| 941 | $CH_3$ | NH—Cyclobutyl | Cl | H | |
| 942 | $CH_3$ | NH—Cyclopentyl | Cl | H | |
| 943 | $CH_3$ | NH—Cycloheptyl | Cl | H | |
| 944 | $CH_3$ | NH—Cyclooctyl | Cl | H | |
| 945 | $CH_3$ | NH—Cyclononyl | Cl | H | |
| 946 | $CH_3$ | NH—Cyclodecyl | Cl | H | |
| 947 | $CH_3$ | NH—Cyclododecyl | Cl | H | |
| 948 | $CH_3$ | $N(CH_3)_2$ | Cl | H | |
| 949 | $CH_3$ | $N(nC_3H_7)_2$ | Cl | H | |
| 950 | $CH_3$ | Pyrrolidino | Cl | H | |
| 951 | $CH_3$ | Piperidino | Cl | H | |

Tabelle 1 (Fortsetzung)

| Bsp. Nr. | Rest $R^1$ | Rest $R^2$ | Rest $R^3$ | Rest $R^4$ | Schmelzpunkt (°C) |
|---|---|---|---|---|---|
| 952 | $CH_3$ | Hexamethylenimino | Cl | H | |
| 953 | $CH_3$ | Morpholino | Cl | H | |
| 954 | $CH_3$ | Thiomorpholino | Cl | H | |
| 955 | $CH_3$ | Thiomorpholino-S,S-dioxid | Cl | H | |

Anwendungsbeispiele

Die folgenden Beispiele zeigen die insektizide Wirkung der Verbindungen I.

Beispiel Nr. 956

Deckel und Boden einer Petrischale wurden mit insgesamt 1 ml einer 0,1 gew.-%igen acetonischen Lösung der Wirkstoffe gleichmäßig ausgekleidet. Nach dem Verdunsten des Lösungsmittels brachte man je 10 Fliegen der Gattung Musca Domestica (Stubenfliege) und Wasserwatte in die Schalen ein. Anschließend wurden die Schalen geschlossen. Nach 24 Stunden wurde die Mortalität der Verbindungen durch Abzählen der gestorbenen Fliegen ermittelt.

Tabelle 2

| Synthese-Bsp. Nr. | Mortalität [%] |
|---|---|
| 12 | 100 |
| 147 | 100 |
| 157 | 80 |
| 155 | 80 |
| 156 | 100 |
| 168 | 100 |
| 395 | 100 |

Beispiel 957

Getopfte Buschbohnen, die das zweite Folgeblattpaar zeigten, einen starken Milbenbefall (Tetranychus telarius, Rote Spinne (A)) und eine reichliche Eiablage aufwiesen, wurden mit 50 ml wäßriger Wirkstofflösung gleichmäßig besprüht. Nach 5 Tagen wurde die Mortalität der Wirkstoffe mittels eines Mikroskops bestimmt. Die Pflanzen standen in dieser Zeit unter üblichen Gewächshausbedingungen.

Tabelle 3

| Synthese-Bsp. Nr. | Substanzkonz. [ppm] | Mortalität [%] |
|---|---|---|
| 155 | 1000 | ca. 90 |
| 156 | ≤ 1000 | 100 |
| 157 | ≤ 1000 | 100 |
| 395 | ≤ 1000 | 100 |

Beispiel 958

Junge Bohnenpflanzen (vicia faba) mit einer starken Kolonie der schwarzen Laus (Aphis fabae) wurden mit je 50 ml einer wäßrigen Lösung des Wirkstoffs gleichmäßig besprüht. Nach 24 Stunden wurde die Mortalität der Wirkstoffe bestimmt.

Tabelle 4

| Synthese-Bsp. Nr. | Substanzkonz. [ppm] | Mortalität [%] |
|---|---|---|
| 154 | ≤ 1000 | 100 |
| 155 | 1000 | 100 |
| 156 | 1000 | 100 |
| 185 | 1000 | 100 |
| 395 | 1000 | 100 |

Beispiel 959

Auf die Oberfläche eines Nährbodens (5 ml in Kunststoffpetrischälchen, Durchmesser 25 mm, Höhe 10 mm) wurden 0,5 ml einer acetonischen Wirkstofflösung gegeben. Nach dem Verdunsten des Acetons wurde der Nährboden mit 30 µl E.-Coli-Bakterien und 50 µl Nematodensuspension (Caenorhabditis elegans) infiziert. Nach 2,8 Stunden wurde die Kontaktwirkung in % Mortalität ermittelt.

Tabelle 5

| Synthese-Bsp. Nr. | Substanzkonz. [ppm] | Mortalität [%] |
|---|---|---|
| 147 | 100 | 100 |
| 154 | 4 | 80 |
| 155 | 4 | 80 |
| 156 | 10 | 100 |
| 157 | 10 | 100 |
| 168 | 100 | 100 |
| 275 | 100 | 100 |
| 282 | 10 | 100 |
| 293 | 100 | 100 |
| 395 | 100 | 100 |

Beispiel 960

Je 5 junge Zecken (Ornithodorus monbata) wurden in einem wasserdurchlässigen Beutel für 5 sec in eine wäßrige Wirkstoffaufbereitung getaucht. Danach wurden die Beutel für 48 Std. frei aufgehängt und anschließend wurde die Mortalität der Wirkstoffe bestimmt.

| Synthese-Beispiel Nr. | Substanzkonz. [ppm] | Mortalität [%] |
|---|---|---|
| 147 | 400 | 100 |
| 154 | 40 | 100 |
| 155 | 1000 | 100 |
| 156 | 400 | 80 |
| 157 | 1000 | 100 |
| 168 | 20 | 80 |

Beispiel 961

Zum Test auf herbizide Wirkungen wurden Samen verschiedener Pflanzen in Plastikblumentöpfe mit lehmigem Sand und 3,0 Gew.-% Humus nach Arten getrennt eingepflanzt. Die Pflanzen wurden bis zu einer Größe von 3 bis 15 cm angezogen und dann einer Nachauflaufbehandlung mit den in Wasser suspendierten Wirkstoffen unterzogen. Die Aufwandmenge betrug 2,0 kg/ha Wirkstoff.

Die in den Gewächshausversuchen verwendeten Pflanzen setzen sich aus folgenden Arten zusammen:

| Abkürzung | Lateinischer Name | Deutscher Name | Englischer Name |
|---|---|---|---|
| AMARE | Amaranthus retroflexus | Zurückgekrümmter Fuchsschwanz | redroot pigweed |
| GALAP | Galium aparine | Klettenlabkraut | catchweed bedstraw |
| LAMAM | Lamium amplexicaule | stengelumfassende Taubnessel | henbit |

Die Versuchsergebnisse wurden nach einer Skala von 0 bis 100 bewertet, wobei 100 bedeutet, daß die Pflanzen völlig zerstört sind und 0 bedeutet das ein normaler Wachstumsverlauf beobachtbar war.

| Synthese-Bsp. Nr. | Testpflanzen und Schädigung | | |
|---|---|---|---|
| | AMARE | GALAP | LAMAM |
| 144 | 100 | 100 | 90 |
| 146 | 100 | 90 | 70 |

## Patentansprüche

1. Thieno[2,3-d]pyrimidinderivate der allgemeinen Formel I

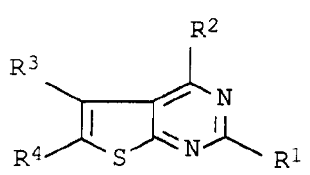

(I)

in der die Substituenten die folgende Bedeutung haben:

$R^1$ Wasserstoff, $C_1$-$C_5$-Alkyl, $C_1$-$C_3$-Chloralkyl, $C_3$-$C_6$-Cycloalkyl, Phenyl oder Benzyl

$R^2$ Fluor, Chlor, Brom, Jod, Hydroxy, Azido, Cyano, Thiocyanato, Mercapto, $C_1$-$C_{20}$-Alkoxy, $C_3$-$C_6$-Alkenyloxy, $C_3$-$C_6$-Alkinyloxy, $C_1$-$C_4$-Alkoxy-$C_2$-$C_6$-alkoxy, Phenoxy-$C_2$-$C_6$-alkoxy, $C_3$-$C_{12}$-Cycloalkoxy, Hydroxy-$C_2$-$C_6$-alkoxy, Chlor-$C_2$-$C_8$-alkoxy, Phenyloxy oder Benzyloxy, Phenylethoxy,

Amino-$C_2$-$C_6$-alkoxy, Bis($C_1$-$C_4$-alkyl)amino-$C_2$-$C_6$-alkoxy, Hydroxy-$C_1$-$C_4$-alkyl-amino-$C_2$-$C_6$-alkoxy, $C_1$-$C_{18}$-Alkylthio, $C_3$-$C_6$-Cycloalkylthio, $C_3$-$C_6$-Alkenylthio, $C_3$-$C_6$-Alkinylthio, Hydroxy-$C_2$-$C_6$-alkylthio, Bis-$C_1$-$C_3$-alkylamino-$C_2$-$C_6$-alkylthio, Carboxy-$C_1$-$C_4$-alkylthio, $C_1$-$C_3$-Alkoxy-carbonyl-$C_1$-$C_2$-alkylthio, Furylmethylthio, Phenylthio oder Benzylthio, $C_1$-$C_4$-Alkoxycarbonyl-methyl, Carboxymethyl, Bis-($C_1$-$C_4$-alkoxycarbonyl)methyl, $\alpha$-$C_1$-$C_4$-Alkoxycarbonyl-$\alpha$-cyano-methyl, Dicyanomethyl, Bis(carboxy)methyl, $\alpha$-Cyano-$\alpha$-phenyl-methyl, Pyrrolidino, Piperidino, Morpholino, Hexamethylenimino, N-Methylpiperazino, N-Ethylpiperazino, Imidazolyl, Pyrazolyl, Triazolyl, Tetrazolyl, Thiomorpholino, Hydrazino, $C_1$-$C_3$-Alkylhydrazino, Bis-($C_1$-$C_3$-alkyl)hydrazino, Phenylhydrazino, Hydroxylamino, Morpholin-(1)-yl-amino, Hexamethylenimino-(1)-yl-amino, oder ein Rest $NR^5R^6$, worin $R^5$ und $R^6$ unabhängig voneinander Wasserstoff, Amino oder $C_1$-$C_{20}$-Alkyl, $C_3$-$C_{20}$-Alkenyl, $C_3$-$C_6$-Alkinyl, $C_3$-$C_{12}$-Cycloalkyl, $C_3$-$C_6$-Cycloalkyl-$C_1$-$C_4$-alkyl, Hydroxy-$C_2$-$C_6$-Alkyl, Amino-$C_2$-$C_{12}$-alkyl, Bis-$C_1$-$C_4$-alkylamino-$C_2$-$C_6$-alkyl, Piperazin-(1)-yl-$C_2$-$C_4$-alkyl, Morpholin-(1)-yl-$C_2$-$C_4$-alkyl, $C_1$-$C_3$-Alkoxy-$C_1$-$C_4$-alkyl, Phenoxy-$C_4$-$C_6$-Alkyl, Benzyloxy-$C_2$-$C_6$-alkyl, Hydroxy-$C_2$-$C_3$-alkoxy-$C_2$-$C_6$-alkyl, Hydroxy-$C_2$-$C_4$-alkylamino-$C_2$-$C_4$-alkyl, $C_2$-$C_3$-Alkoxy-$C_2$-$C_3$-alkoxy-$C_2$-$C_6$-alkyl, Phenoxy-$C_2$-$C_4$-alkoxy-$C_2$-$C_4$-alkyl, Bis-hydroxy-methyl-$C_1$-$C_3$-alkyl , Cyano-$C_2$-$C_{10}$-alkyl, Carboxy-$C_2$-$C_{10}$-alkyl, $C_1$-$C_3$-Alkoxy-$C_2$-$C_3$alkoxy-$C_2$-$C_3$-alkoxy-$C_2$-$C_3$-alkyl, Phenyl, Phenyl-$C_1$-$C_4$-alkyl, $C_3$-$C_{12}$-Cycloalkyl, Pyridyl, Furyl, Thienyl, Methylpyridyl, Pyridylmethyl, Furylmethyl, Thienylmethyl oder Adamantyl bedeuten oder gemeinsam mit dem Stickstoffatom einen heterocyclischen Rest mit 3 bis 8 Ringatomen bilden, mit der Maßgabe, daß $R^2$ nicht Phenoxy-$C_2$-$C_3$-alkylamino bedeutet,

$R^3$ Chlor, Brom, Hydroxy oder Mercapto mit der Maßgabe, daß nicht gleichzeitig $R^2$ Hydroxy und R3 Brom bedeutet und daß nicht gleichzeitig $R^3$ Hydroxy und R4 Alkoxycarbonyl oder Carboxy bedeutet,

$R^4$ Wasserstoff, $C_1$-$C_6$-Alkyl, $C_3$-$C_6$-Mono-, Di- oder Trihalogenalkyl, Phenyl, Cyano, Formyl, Hydroxyiminomethyl, Carboxy, Alkoxycarbonyl, Aminocarbonyl oder ein Rest der Formel

CH=X

worin X für $C_1$-$C_{20}$-Alkylimino, Phenylimino, Carboxymethylen, $\alpha$-$C_1$-$C_4$-Alkyl-$\alpha$-carboxymethylen, $\alpha$-Phenyl-$\alpha$-carboxymethylen, Dicarboxymethylen, Cyanomethylen, $\alpha$-$C_1$-$C_4$-Alkyl-$\alpha$-cyanomethylen, $\alpha$-Phenyl-$\alpha$-cyanomethylen, Dicyanomethylen, Formylmethylen, $\alpha$-$C_1$-$C_4$-Alkyl-$\alpha$-formylmethylen, $\alpha$-Phenyl-$\alpha$-formylmethylen, Diformylmethylen, $C_1$-$C_4$-Alkoxycarbonylmethylen, $\alpha$-$C_1$-$C_4$-Alkyl-$\alpha$-$C_1$-$C_4$-Alkoxycarbonylmethylen, $C_1$-$C_4$-Di-alkoxycarbonylmethylen, $\alpha$-Cyano-$\alpha$-$C_1$-$C_4$-alkoxycarbonylmethylen oder $\alpha$-Phenyl-$\alpha$-$C_1$-$C_4$-alkoxycarbonylmethylen steht,

wobei, wenn $R^1$ und/oder $R^2$ Phenyl bedeuten oder enthalten, dieser Phenylrest bis zu 3 gleiche oder verschiedene Substituenten, wie $C_1$-$C_4$-Alkyl, $C_1$-$C_4$-Alkoxy, Halogen, Nitro, Amino, Hydroxy, Hydroxy-$C_1$-$C_4$-alkyl, Cyano, Carboxy, Aminocarbonyl, $C_1$-$C_4$-Alkoxycarbonyl, $C_1$-$C_4$-Acyloxy, $C_2$-$C_6$-Acylaminol $C_1$-$C_4$-Alkylamino, $C_1$-$C_3$-Acyl, Carboxy-$C_1$-$C_2$-alkyl, Di-$C_1$-$C_4$-alkylamino sowie Trifluormethyl tragen kann, sowie die Tautomeren dieser Thieno[2,3-d]pyrimidinderivate und die nutzpflanzenverträglichen Salze dieser Thieno[2,3-d]pyrimidinderivate und ihrer Tautomeren.

**2.** Verfahren zur Herstellung von Thieno[2,3-d]pyrimidinderivaten der allgemeinen Formel I nach Anspruch 1, dadurch gekennzeichnet, daß man Verbindungen der allgemeinen Formel II

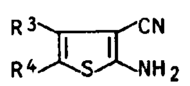

(II)

in der $R^3$ und $R^4$ die in Anspruch 1 angegebene Bedeutung haben, mit einer Verbindung der allgemeinen Formel III

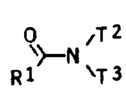

(III)

in der $R^1$ die in Anspruch 1 angegebene Bedeutung hat und $T^2$ sowie $T^3$ unabhängig voneinander für $C_1$-$C_4$-Alkyl, Phenyl, oder zusammen mit dem Amidstickstoff für einen 5 bis 7 gliedrigen gesättigten Heterocyclus stehen, in Gegenwart von 1,1 bis 20 mol Phosphorylchlorid oder Phosphorylbromid, bezogen auf ein Mol II, zu Thieno[2,3-d]pyrimidinderivaten der allgemeinen Formel Ia

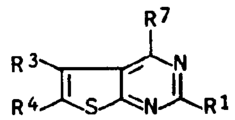

(Ia)

in der $R^7$ für Chlor oder Brom steht, umsetzt und in diesen gegebenenfalls $R^7$ in an sich üblicher Weise gegen andere nucleophile Reste $R^2$ austauscht.

3. Verfahren zur Herstellung von Thieno[2,3-d]pyrimidinderivaten der allgemeinen Formel I nach Anspruch 1, dadurch gekennzeichnet, daß man Verbindungen der allgemeinen Formel IV

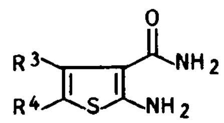

(IV)

in der die Substituenten $R^3$ und $R^4$ die in Anspruch 1 angegebene Bedeutung haben, in an sich üblicher Weise mit einem Säureanhydrid, das mindestens einen Rest $R^1$-CO- enthält, oder einer Carbonsäure $R^1$-COOH, oder einem Addukt aus einer Carbonsäure $R^1$-COOH und einer Lewissäure, wobei $R^1$ jeweils die in Anspruch 1 angegebene Bedeutung hat, zu Verbindungen der allgemeinen Formel Ib

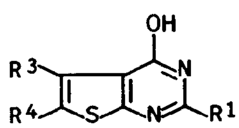

(Ib)

umsetzt, diese mit einem Phosphorylhalogenid in Verbindungen der allgemeinen Formel Ia gemäß Anspruch 2 überführt und in diesen gegebenenfalls $R^7$ in an sich üblicher Weise gegen andere nucleophile Reste $R^2$ austauscht.

4. Verfahren zur Herstellung von Thieno[2,3-d]pyrimidinen der allgemeinen Formel I nach Anspruch 1, dadurch gekennzeichnet, daß man ein Thiophen der allgemeinen Formel II

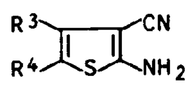

(II)

in der $R^3$ und $R^4$ die in Anspruch 1 angegebene Bedeutung haben, in an sich bekannter Weise mit Orthoestern der allgemeinen Formel V

$$(R^8O)_3C\text{-}R^1 \qquad (V)$$

in der $R^8$ für einen $C_1$-$C_6$-Alkylrest steht, zu Verbindungen der allgemeinen Formel VI

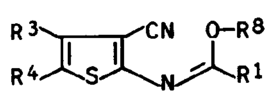

(VI)

umsetzt und diese in an sich bekannter Weise in Gegenwart eines den Rest $R^2$ enthaltenden Nucleophils in die entsprechenden Verbindungen I überführt.

5. Herbizide Mittel, enthaltend einen Trägerstoff und eine herbizid wirksame Menge eines Thieno[2,3-d]pyrimidinderivates der allgemeinen Formel I gemäß Anspruch 1.

6. Schädlingsbekämpfungsmittel, enthaltend einen Trägerstoff und eine wirksame Menge eines Thieno[2,3-d]pyrimidinderivates der allgemeinen Formel I gemäß Anspruch 1.

7. Wachstumsregulierende Mittel, enthaltend einen Trägerstoff und eine wachstumsregulierende Menge eines Thieno[2,3-d]pyrimidinderivates der allgemeinen Formel I gemäß Anspruch 1.

8. Verwendung von Thieno[2,3-d]pyrimidinen der allgemeinen Formel I gemäß Anspruch 1 zur Bekämpfung von Schadpflanzen.

9. Verwendung von Thieno[2,3-d]pyrimidinen der allgemeinen Formel I gemäß Anspruch 1 zur Bekämpfung von Schädlingen.

10. Verwendung von Thieno[2,3-d]pyrimidinen der allgemeinen Formel I gemäß Anspruch 1 zur Regulation des Nutzpflanzenwachstums.

## Claims

1. A thieno[2,3-d]pyrimidine derivative of the formula I

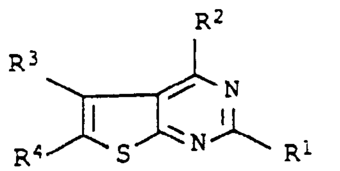

(I)

where

$R^1$ is hydrogen, $C_1$-$C_5$-alkyl, $C_1$-$C_3$-chloroalkyl, $C_3$-$C_6$-cycloalkyl, phenyl or benzyl,

$R^2$ is fluorine, chlorine, bromine, iodine, hydroxyl, azido, cyano, thiocyanato, mercapto, $C_1$-$C_{20}$-alkoxy, $C_3$-$C_6$-alkenyloxy, $C_3$-$C_6$-alkynyloxy, $C_1$-$C_4$-alkoxy-$C_2$-$C_6$- alkoxy, phenoxy-$C_2$-$C_6$-alkoxy, $C_3$-$C_{12}$-cycloalkoxy, hydroxy-$C_2$-$C_6$-alkoxy, chloro-$C_2$-$C_8$-alkoxy, phenyloxy or benzyloxy, phenylethoxy, amino-$C_2$-$C_6$-alkoxy, bis($C_1$-$C_4$-alkyl)amino-$C_2$-$C_6$-alkoxy, hydroxy-$C_1$-$C_4$-alkylamino-$C_2$-$C_6$-alkoxy, $C_1$-$C_{18}$-alkylthio, $C_3$-$C_6$-cycloalkylthio, $C_3$-$C_6$-alkenylthio, $C_3$-$C_6$-alkynylthio, hydroxy-$C_2$-$C_6$-alkylthio, bis -$C_1$-$C_3$-alkylamino-$C_2$-$C_6$-alkylthio, carboxy-$C_1$-$C_4$-alkylthio, $C_1$-$C_3$-alkoxycarbonyl-$C_1$-$C_2$-alkylthio, furylmethylthio, phenylthio or benzylthio, $C_1$-$C_4$-alkoxycarbonylmethyl, carboxymethyl, bis($C_1$-$C_4$-alkoxycarbonyl)methyl, α-$C_1$-$C_4$-alkoxycarbonyl-α-cyanomethyl, dicyanomethyl, bis(carboxy)methyl, α-cyano-α-phenylmethyl, pyrrolidino, piperidino, morpholino, hexamethyleneimino, N-methylpiperazino, N-ethylpiperazino, imidazolyl, pyrazolyl, triazolyl, tetrazolyl, thiomorpholino, hydrazino, $C_1$-$C_3$-alkylhydrazino, bis($C_1$-$C_3$-alkyl)hydrazino, phenylhydrazino, hydroxylamino, morpholinoamino or hexamethyleneiminoamino, or $NR^5R^6$ where $R^5$ and $R^6$ are each, independently of one another, hydrogen, amino or $C_1$-$C_{20}$-alkyl, $C_3$-$C_{20}$-alkenyl, $C_3$-$C_6$-alkynyl, $C_3$-$C_{12}$-cycloalkyl, $C_3$-$C_6$-cycloalkyl-$C_1$-$C_4$-alkyl, hydroxy-$C_2$-$C_6$-alkyl, amino-$C_2$-$C_{12}$-alkyl, bis-$C_1$-$C_4$-alkylamino-$C_2$-$C_6$-alkyl, piperazino-$C_2$-$C_4$-alkyl, morpholino-$C_2$-$C_4$-alkyl, $C_1$-$C_3$-alkoxy-$C_1$-$C_4$-alkyl, phenoxy-$C_4$-$C_6$-alkyl, benzyloxy-$C_2$-$C_6$-alkyl, hydroxy-$C_2$-$C_3$-alkoxy-$C_2$-$C_6$-alkyl, hydroxy-$C_2$-$C_4$-alkylamino-$C_2$-$C_4$-alkyl, $C_2$-$C_3$-alkoxy-$C_2$-$C_3$-alkoxy-$C_2$-$C_6$-alkyl, phenoxy-$C_2$-$C_4$-alkoxy-$C_2$-$C_4$-alkyl, bis-hydroxymethyl-$C_1$-$C_3$-alkyl, cyano-$C_2$-$C_{10}$-alkyl, carboxy-$C_2$-$C_{10}$-alkyl, $C_1$-$C_3$-alkoxy-$C_2$-$C_3$-alkoxy-$C_2$-$C_3$-alkoxy-$C_2$-$C_3$-alkyl, phenyl, phenyl-$C_1$-$C_4$-alkyl, $C_3$-$C_{12}$-cycloalkyl, pyridyl, furyl, thienyl, methylpyridyl, pyridylmethyl, furylmethyl, thienylmethyl or adamantyl, or they form, together with the nitrogen atom, a heterocyclic radical with 3 to 8 ring atoms, with the proviso that $R^2$ is not phenoxy-$C_2$-$C_3$-alkylamino,

$R^3$ is chlorine, bromine, hydroxyl or mercapto, with the proviso that $R^2$ is not hydroxyl when $R^3$ is bromine, and that $R^3$ is not hydroxyl when $R^4$ is alkoxycarbonyl or carboxyl,

$R^4$ is hydrogen, $C_1$-$C_6$-alkyl, $C_3$-$C_6$-mono-, di- or tri- haloalkyl, phenyl, cyano, formyl, hydroxyiminomethyl, carboxyl, alkoxycarbonyl, aminocarbonyl or a radical of the formula

$$CH{=}X$$

where X is $C_1$-$C_{20}$-alkylimino, phenylimino, carboxymethylene, α-$C_1$-$C_4$-alkyl-α-carboxymethylene, α-phenyl-α-carboxymethylene, dicarboxymethylene, cyanomethylene, α-$C_1$-$C_4$-alkyl-α-cyanomethylene, α-phenyl-α-cyanomethylene, dicyanomethylene, formylmethylene, α-$C_1$-$C_4$-alkyl-α-formylmethylene, α-phenyl-α-formylmethylene, diformylmethylene, $C_1$-$C_4$-alkoxycarbonylmethylene, α-$C_1$-$C_4$-alkyl-α-$C_1$-$C_4$-alkoxycarbonylmethylene, $C_1$-$C_4$-dialkoxycarbonylmethylene, α-cyano-α-$C_1$-$C_4$-alkoxycarbonylmethy-

lene or α-phenyl-α-$C_1$-$C_4$-alkoxycarbonylmethylene, it being possible, when $R^1$ and/or $R^2$ is or contains phenyl, for this phenyl to carry up to 3 identical or different substituents such as $C_1$-$C_4$-alkyl, $C_1$-$C_4$-alkoxy, halogen, nitro, amino, hydroxyl, hydroxy-$C_1$-$C_4$-alkyl, cyano, carboxyl, aminocarbonyl, $C_1$-$C_4$-alkoxycarbonyl, $C_1$-$C_4$-acyloxy, $C_2$-$C_6$-acylamino, $C_1$-$C_4$-alkylamino, $C_1$-$C_3$-acyl, carboxy-$C_1$-$C_2$-alkyl, di-$C_1$-$C_4$-alkylamino and trifluoromethyl,
tautomers thereof, and salts and tautomers thereof which are tolerated by crop plants.

2. A process for preparing a thieno[2,3-d]pyrimidine derivative of the formula I as claimed in claim 1, which comprises reacting a compound of the formula II

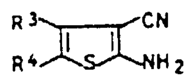

(II)

where $R^3$ and $R^4$ have the meanings indicated in claim 1, with a compound of the formula III

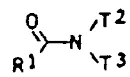

(III)

where $R^1$ has the meanings indicated in claim 1 and $T^2$ and $T^3$ are each, independently of one another, $C_1$-$C_4$-alkyl or phenyl or form, together with the amide nitrogen, a 5- to 7-membered saturated heterocycle, in the presence of from 1.1 to 20 mol of phosphoryl chloride or phosphoryl bromide per mol of II to give a thieno[2,3-d]pyrimidine derivative of the formula Ia

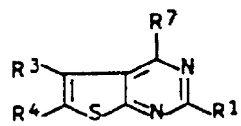

(Ia)

where $R^7$ is chlorine or bromine, and replacing $R^7$ in this where appropriate by other nucleophilic radicals $R^2$ in a conventional manner.

3. A process for preparing a thieno[2,3-d]pyrimidine derivative of the formula I as claimed in claim 1, which comprises reaching a compound of the formula IV

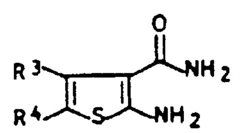

(IV)

where $R^3$ and $R^4$ have the meanings indicated in claim 1, in a conventional manner with an anhydride which contains at least one $R^1$-CO- radical, or with a carboxylic acid $R^1$-COOH, or with an adduct of a carboxylic acid $R^1$-COOH and a Lewis acid, where $R^1$ has the stated meanings, to give a compound of the formula 1b

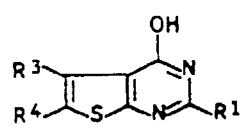

(Ib)

converting the latter with a phosphoryl halide into a compound of the formula Ia as claimed in claim 2, and replacing $R^7$ in the latter where appropriate by other nucleophilic radicals $R^2$ in a conventional manner.

4. A process for preparing a thieno[2,3-d]pyrimidine of the formula I as claimed in claim 1, which comprises reacting a thiophene of the formula II

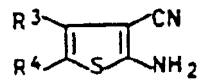

(II)

where $R^3$ and $R^4$ have the meanings indicated in claim 1, in a conventional manner with an orthoester of the formula V

$$(R^8O)_3C\text{-}R^1 \qquad (V)$$

where $R^8$ is $C_1$-$C_6$-alkyl, to give a compound of the formula VI

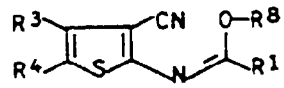

(VI)

and converting the latter in a conventional manner into a corresponding compound I in the presence of a nucleophile containing $R^2$.

5. A herbicide containing a carrier and a herbicidally effective amount of a thieno[2,3-d]pyrimidine derivative of the formula I as claimed in claim 1.

6. A pesticide containing a carrier and an effective amount of a thieno[2,3-d]pyrimidine derivative of the formula I as claimed in claim 1.

7. A growth regulator containing a carrier and a growth-regulating amount of a thieno[2,3-d]pyrimidine derivative of the formula I as claimed in claim 1.

8. The use of a thieno[2,3-d]pyrimidine of the formula I as claimed in claim 1 for controlling unwanted plants.

9. The use of a thieno[2,3-d]pyrimidine of the formula I as claimed in claim 1 for controlling pests.

10. The use of a thieno[2,3-d]pyrimidine of the formula I as claimed in claim 1 for regulating the growth of crop plants.

## Revendications

1. Dérivé de thiéno (2,3-d)pyrimidine de la formule générale I

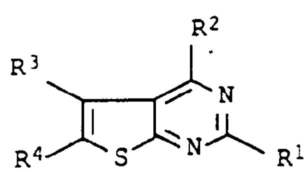

(I)

dans laquelle les substituants ont la signification suivante :
$R^1$ hydrogène, alkyle en C1-C5, chloroalkyle en C1-C3, cycloalkyle en C3-C6, phényle ou benzyle
$R^2$ fluor, chlore, brome, iode, hydroxy, azido, cyano, thiocyanato, mercapto, alcoxy en C1-C20, alcényloxy en C3-C6, alcinyloxy en C3-C6, alcoxy en C1-C4-alcoxy en C2-C6, phénoxy-alcoxy en C2-C6, cyclo-alcoxy en C3-C12, hydroxy-alcoxy en C2-C6, chlore-alcoxy en C2-C8, phényloxy ou benzyloxy, phényléthoxy, amino-alcoxy en C2-C6, bis (alkyle en C1-C4)amino-alcoxy en C2-C6, hydroxy-alkyle en C1-C4-amino-alcoxy en C2-C6, alkylthio en C1-C18, cycloalkylthio en C3-C6, alcényle-thio en C3-C6, alcinyle-thio en C3-C6, hydroxy-alkyle-thio en C2-C6, bis-alkyle-amino en C1-C3-alkyle-thio en C2-C6, carboxy-alkyle-thio en C1-C4, alcoxy-carbonyle en C1-C3-alkyle-thio en C1-C2, furylméthylthio, phénylthio ou benzylthio, alcoxy-carbonylméthyle en C1-C4, carboxyméthyle, bis-(alcoxy-carbonyle en C1-C4)méthyle, α-alcoxy- carbonyle en C1-C4-α-cyanométhyle, dicyanométhyle, bis(carboxy)méthyle, α-cyano-α-phényle-méthyle, pyrrolidino, pipéridino, mopholino, hexaméthylénimino, N-méthylpipérazino, N-éthyle-pipérazino, imidazolyle, pyrazolyle, triazolyle, tétrazolyle, thiomorpholino, hydrazino, alkylhydrazino en C1-C3, bis-(alkyle en C1-C3)hydrazino, phénylhydrazino, hydroxylamino, morpholine-(1)-yl-amino, hexa-

méthylenimino-(1)-yl-amino, ou un reste $NR^5R^6$, ou $R^5$ et $R^6$ indépendant l'un de l'autre hydrogène, amino ou alkyle en C1-C20, alcényle en C3-C20, alcinyle en C3-C6, cycloalkyle en C2-C12, cycloalkyle en C3-C6-alkyle en C1-C4, hydroxy-alkyle en C2-C6, amino-alkyle en C2-C12, bis-alkyle-amino en C1-C4-lakyle en C2-C6, pipérazine-(1)-yl-alkyle en C2-C4, morpholine-(1)-yl-alkyle en C2-C4, alcoxy en C1-C3-alkyle en C1-C4, phénoxy-alkyle en C4-C6, benzyloxy-alkyle en C2-C6, hydroxy-alcoxy en C2-C3-alkyle en C2-C6, hydroxy-alkyle-amino en C2-C4-alkyle en C2-C4, alcoxy en C2-C3-alcoxy en C2-C3-alkyle en C2-C6, phénoxy-alcoxy en C2-C4-alkyle en C2-C4, bis-hydroxyméthyle-alkyle en C1-C3, cyano-alkyle en C2-C10, carboxy-alkyle en C2-C10, alcoxy en C1-C3-alcoxy en C2-C3-alcoxy en C2-C3-alkyle en C2-C3, phényle, phényle-alkyle en C1-C4, cycloalkyle en C3-C12, pyridyle, furyle, thiényle, méthylpyridyle, pyridylméthyle, furylméthyle, thienylméthyle ou adamantyle ou bien forment ensemble avec l'atome d'azote un reste hétérocyclique de 3 à 8 atomes de cycle, sous réserve que $R^2$ ne représente pas phénoxy-(alkyle en C2-C3)amino.

$R^3$ représente chlore, brome, hydroxy ou mercapto sous réserve que $R^2$ ne représente pas hydroxy quand $R^3$ représente brome et que $R^3$ ne représente pas hydroxy quand $R^4$ représente alcoxycarbonyle ou carboxy.

$R^4$ est mis pour hydrogène alkyle en C1-C6, -mono-, di- ou trihalogéalkyle en C3-C6, phényle, cyano, formyle, hydroxyiminométhyle, carboxy,alcoxycarbonyle, aminocarbonyle ou un reste de la formule

CH=X

où X est mis pour alkyle-imino en C1-C20, phénylimino, carboxyméthylène, α-alkyle en C1-C4-α-carboxyméthylène, α-phényle-α-carboxyméthylène, dicarboxyméthylène, cyanométhylène, α-alkyle en C1-C4-α-cyanométhyléne, α-phényle-α-cyanométhylène, dicyanométhylène, formylméthylène, α-alkyle en C1-C4-α-formylméthylène, α-phényle-α-formylméthylène, diformylméthylène, alcoxy-carbonylméthylène en C1-C4, α-alkyle en C1-C4-α-alcoxy-carbonylméthylène en C1-C4, di-alcoxy-carbonylméthylène en C1-C4, α-cyano-α-alcoxy-carbonylméthyléne en C1-C4 ou α-phényle-α-alcoxy-carbonylméthyléne en C1-C4,

ce reste phényle, quand $R^1$ et/ou $R^2$ représentent ou contiennent phényle, pouvant porter jusqu'à 3 substituants identiques ou différents, tels que alkyle en C1-C4, alcoxy en C1-C4, halogène, nitro, amino, hydroxy, hydroxy-alkyle en C1-C4, cyano, carboxy, aminocarbonyle, alcoxy en C1-C4-carbonyle, acyloxy en C1-C4,acylamino en C2-C6, alkyle-amino en C1-C4, acyle en C1-C3, carboxy-alkyle en C1-C2, di-alkyle-amino en C1-C4 ainsi que trifluorométhyle, ainsi que les tautomères de ces dérivés de thiéno (2,3-d)pyrimidine et les sels acceptables pour les plantes utiles de ces dérivés de thiéno(2,3-d)pyrimidine et leurs tautomères.

2. Procédé de préparation de dérivés de thiéno (2,3-d)pyrimidine de la formule générale I selon la revendication 1, caractérisé par le fait qu'on fait réagir des composés de formule générale II

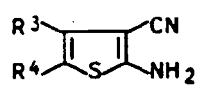

(II)

dans laquelle $R^3$ et $R^4$ ont la signification donnée dans la revendication 1 avec un composé de la formule générale III

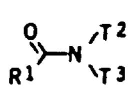

(III)

dans laquelle $R^1$ a la signification donnée dans la revendication 1 et $T^2$ et $T^3$ indépendamment l'un de l'autre, sont mis pour alkyle en C1-C4, phényle, ou ensemble avec l'azote d'amide pour un hétérocyle saturé a 5 à 7 chaînons, en présence de 1,1 à 20 mol de chlorure ou bromure de phosphonyle, rapporté à une mol II, pour obtenir des dérivés de thiéno[2,3-d]pyrimidine de la formule générale Ia

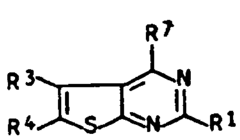

(Ia)

dans laquelle $R^7$ est mis pour chlore ou brome, et dans celui-ci, on échange éventuellement $R^7$ de manière connue en soi, contre d'autres restes $R^2$ nucléophiles.

3. Procédé de préparation de dérivés de thiéno( 2,3-d)pyrimidine de la formule générale I selon la revendication 1, caractérisé par le fait que l'on fait réagir de manière connue en soi des composés de la formule générale IV

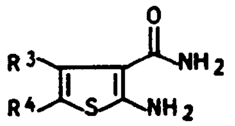

(IV)

dans laquelle les subsituants $R^3$ et $R^4$ ont la signification donnée dans la revendication 1, avec un anhydre d'acide qui contient au moins un reste $R^1$-CO-, ou un acide carboxylique $R^1$-COOH, ou un adduct d'un acide carboxylique $R^1$-COOH et d'un acide de Lewis, $R^1$ ayant chaque fois, la signification donnée dans la revendication 1, pour obtenir des composés de la formule générale Ib

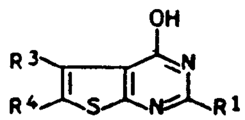

(Ib)

on transforme ceux-ci, avec un halogénure de phosphoryle en composés de la formule générale la selon la revendication 2 et dans ceux-ci, on échange éventuellement $R^7$, de manière connue en soi, contre d'autres reste $R^2$ nucléophiles.

4. Procédé de préparation de dérivés de thiéno(2,3-d)pyrimidine de la formule générale I selon la revendication 1, caractérisé par le fait que l'on fait réagir de manière connue en soi, un thiophène de formule générale II

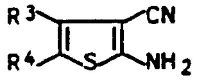

(II)

dans laquelle $R^3$ et $R^4$ ont la signification donnée dans la revendication 1 avec des orthoesters de la formule générale V

$$(R^8O)_3C\text{-}R^1 \qquad (V)$$

dans laquelle $R^8$ est mis pour un reste alkyle en C1-C6, pour obtenir des composés de la formule générale VI

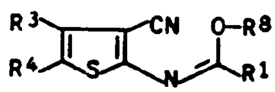

(VI)

et on transforme de manière connue, ceux-ci en présence d'un nucléophile contenant le reste $R^2$, en les composés I correspondants.

5. Agent herbicide contenant un support et une quantité à activité herbicide d'un dérivé de thiéno[2,3-d]pyrimidine de la formule générale I selon la revendication 1.

6. Agent de lutte contre les pesticides, contenant un support et une quantité à activité herbicide d'un dérivé de thiéno[2,3-d]pyrimidine de la formule générale I selon la revendication 1.

7. Agent de régularisation de la croissance contenant un support et une quantité à activité herbicide d'un dérivé de thiéno[2,3-d]pyrimidine de la formule générale I selon la revendication 1.

8. Utilisation de thiéno[2,3-d]pyrimidines de la formule générale I, selon la revendication 1 pour lutter contre des pesticides.

9. Utilisation de thiéno[2,3-d]pyrimidines de la formule générale I, selon la revendication 1 pour lutter contre les nuisibles.

10. Utilisation de thiéno[2,3-d]pyrimidines de la formule générale I, selon la revendication 1 pour la régularisation de la croissance des plantes utiles.